(19) Europäisches Patentamt
European Patent Office
Office européen des brevets

(11) EP 2 933 275 A1

(12) EUROPEAN PATENT APPLICATION

(43) Date of publication:
**21.10.2015 Bulletin 2015/43**

(51) Int Cl.:
***C08F 210/16*** *(2006.01)* ***C08F 4/6592*** *(2006.01)*
***C07F 15/00*** *(2006.01)*

(21) Application number: **14165140.6**

(22) Date of filing: **17.04.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Borealis AG**
**1220 Vienna (AT)**

(72) Inventors:
- **Ajellal, Noureddine**
  **00970 Helsinki (FI)**
- **Pellecchia, Roberta**
  **4040 Linz (AT)**
- **Resconi, Luigi**
  **44100 Ferrara (IT)**

(74) Representative: **Lindinger, Ingrid**
**Borealis Polyolefine GmbH**
**St.-Peter-Straße 25**
**4021 Linz (AT)**

# (54) New catalyst system for producing polyethylene copolymers in a high temperature solution polymerization process

(57) Catalyst system for producing ethylene copolymers in a high temperature solution process, the catalyst system comprising
(i) a metallocene complex of formula (I)

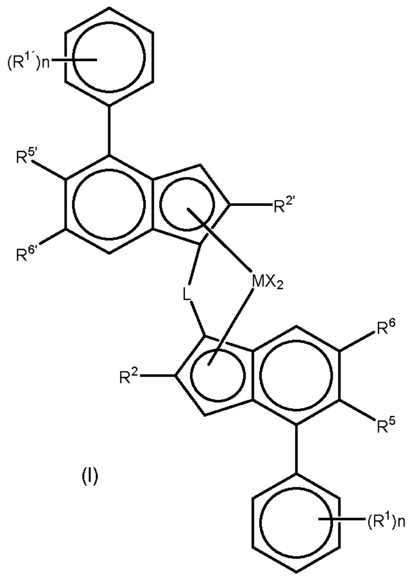

(I)

wherein
M is Hf or Zr
X is a sigma ligand
L is a bridge of the formula $-SiR^7_2-$, wherein each $R^7$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl
$R^1$ and $R^{1'}$ are the same or can be different and can be a linear or branched $C_1$-$C_6$-alkyl group
n is 0, 1 or 2
$R^2$ and $R^{2'}$ are the same or can be different and are a $CH_2$-$R^8$ group, with $R^8$ being H or linear or branched $C_1$-$C_6$-alkyl group
$R^5$ and $R^{5'}$ are the same or are different and can be a linear or branched $C_1$-$C_6$-alkyl group or a OR group, wherein R is a $C_1$-$C_6$-alkyl group
$R^6$ and $R^{6'}$ are the same or are different and can be a C$(R^9)_3$ group, with $R^9$ being the same or different and $R^9$ can be H or a linear or branched $C_1$-$C_6$-alkyl group
or $R^5$ and $R^6$ and/or $R^{5'}$ and $R^{6'}$ taken together form an unsubstituted 4-7 membered ring condensed to the benzene ring of the indenyl moiety,
with the proviso that if $R^5$ and $R^6$ as well as $R^{5'}$ and $R^{6'}$ taken together form an unsubstituted 5 membered ring condensed to the benzene ring of the indenyl moiety then $R^2$ and $R^{2'}$ are not a $CH_3$ group

(ii) an aluminoxane cocatalyst and
(iii) optionally a boron containing cocatalyst

## Description

The present invention is related to a new catalysts system, which is able to produce polyethylene copolymers in a high temperature solution polymerization process. The new catalyst system comprises a special bisindenyl metallocene complex, substituted in position 2, 4, 5 and 6 on both indenyls, along with an aluminoxane cocatalyst and optionally additionally a boron based cocatalyst. This combination remarkably gives rise to catalyst systems with excellent activity, productivity and stability and allows production of polyethylene copolymers with increased excellent comonomer incorporation.

Metallocene catalysts have been used to manufacture polyolefins for many years. Countless academic and patent publications describe the use of these catalysts in olefin polymerization. Metallocenes are now used industrially and polyethylenes and in particular polypropylenes are often produced using cyclopentadienyl based catalyst systems with different substitution patterns.

Several of these metallocene catalysts have been described for the use in solution polymerization in particular for producing polypropylene. For example WO 2007/116034 describes i.a. a catalyst system comprising racemic dimethylsilylbis(2-methyl-4-phenyl-5-methoxy-6-tert-butylinden-1-yl)dichlorozirconium and methylalumoxane cocatalyst for producing polypropylene in a solution polymerization process at temperatures between 100°C and 120°C.

It is mentioned that the metallocene compounds can also be used for preparing ethylene copolymers, preferably ethylene-butene copolymers, but it is said that such copolymers are obtained by using gas phase processes.

Also WO 2007/122098 describes the use of the complex racemic dimethylsilylbis(2-methyl-4-(4-tert-butylphenyl)-1,5,6,7-tetrahydro-s-indacen-1-yl)dichlorozirconium in combination with an alumoxane cocatalyst for producing ethylene copolymers at 100°C.

EP 2532687 A describes further metallocene complexes, like dimethylsilanediylbis[2-methyl-4-(3,5-di-*tert*-butylphenyl)-7-methoxy-indenyl]zirconiumdichloride, which is first pre-alkylated with an aluminium alkyl compound and then activated with borate cocatalyst. The catalyst system is used for preparing polypropylene at a temperature between 30°C to 70°C.

WO 2011/135004 complexes as described in WO 2007/116034, like racemic dimethylsilylbis(2-methyl-4-phenyl-5-methoxy-6-tert-butylinden-1-yl)dichlorozirconium and prepared according to the emulsion/solidification method as described in WO 2003/051934 are disclosed. These complexes are activated with an alumoxane cocatalyst and used for propylene polymerization.

WO 2012/075560 further describes a multi stage (at least two stage) solution polymerization process for preparing ethylene copolymers, wherein a phosphinimine catalyst is used with a cocatalyst comprising an alkylaluminoxane and an ionic activator, like a boron compound.

In none of the above cited literatures the problem of effective comonomer incorporation is mentioned.

However, for a process for producing ethylene copolymers to be efficient, it is important that the catalyst system used has a high reactivity for the C4-10 alpha-olefins used as comonomer.

Drawbacks arising from a low reactivity for the C4-10 alpha-olefin comonomer are e.g. increasing amounts of the alpha-olefin comonomer that are needed for introducing a certain amount of higher alpha-olefin comonomer units into the polymer and/or removal of non-reacted higher alpha-olefin from the polymer powder.

A further important and desired property of the catalyst system used is a high productivity in order to get a maximum of polyethylene produced with as low amount of catalyst as possible. One further point to be noted is that high-temperature solution processes for olefin polymerization require a thermally robust catalyst.

As is discussed in WO 2003/102042 solution processes are characterized by short residence times. Consequently, in addition to having temperature stability, the catalyst systems used in these processes must activate quickly and thoroughly. This contrasts sharply with the requirements for catalysts used in slurry and gas-phase processes, where residence times are longer and catalyst lifetime is more important. Thus, a catalyst that is valuable for slurry and gas-phase processes might be a poor choice for use in a high-temperature solution process, and vice-versa. As solution to this problem WO 2003/102042 suggests to use organometallic complexes having Group 3-10 transition metal and a bridged indeno-indolyl ligand in combination with an activator, which is preferably methylalumoxane.

Although a lot of work has been done in the field of metallocene catalysts, there still remains a need to find new catalyst systems for ethylene copolymerization, which are able to produce polymers with desired properties and which have high productivity, high reactivity for the used comonomers in order to achieve high comonomer incorporation and high thermal stability.

As a consequence, the inventors set out to develop a new catalyst system having superior polymerization behaviour than the above mentioned polymerization catalyst systems regarding to productivity, comonomer incorporation and thermal stability.

The present inventors have now found a new class of olefin polymerization catalyst systems, which are able to solve the problems disclosed above. In particular, the invention combines the use of special metallocene complexes

with aluminoxane cocatalysts and optionally in addition a boron cocatalyst.

**Summary of Invention**

Thus, viewed from one aspect the invention relates to a catalyst system for producing ethylene copolymers in a high temperature solution process, the catalyst system comprising

(i) a metallocene complex of formula (I)

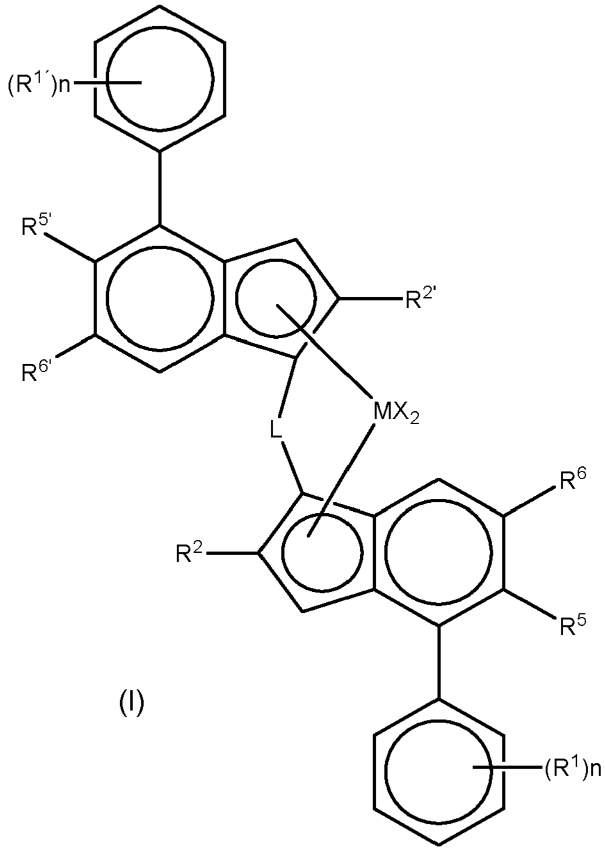

(I)

wherein

M is Hf or Zr
X is a sigma ligand
L is a bridge of the formula $-SiR^7{}_2-$, wherein each $R^7$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl
$R^1$ and $R^{1'}$ are the same or can be different and can be a linear or branched $C_1$-$C_6$-alkyl group
n is 0, 1 or 2
$R^2$ and $R^{2'}$are the same or can be different and are a $CH_2$-$R^8$ group, with $R^8$ being H or linear or branched $C_1$-$C_6$-alkyl group
$R^5$ and $R^{5'}$ are the same or are different and can be a linear or branched $C_1$-$C_6$-alkyl group or a OR group, wherein R is a $C_1$-$C_6$-alkyl group
$R^6$ and $R^{6'}$ are the same or are different and can be a $C(R^9)_3$ group, with $R^9$ being the same or different and $R^9$ can be H or a linear or branched $C_1$-$C_6$-alkyl group
or $R^5$ and $R^6$ and/or $R^{5'}$ and $R^{6'}$ taken together form an unsubstituted 4-7 membered ring condensed to the benzene ring of the indenyl moiety,

with the proviso that if $R^5$ and $R^6$ as well as $R^{5'}$ and $R^{6'}$ taken together form an unsubstituted 5 membered ring condensed to the benzene ring of the indenyl moiety then $R^2$ and $R^{2'}$ are not a $C_1$-alkyl group;
(ii) an aluminoxane cocatalyst and

(iii) optionally a boron containing cocatalyst

Viewed from another aspect the invention provides a new class of metallocenes of formula (I), wherein
M is Zr,
X is Cl or methyl group,
L is a bridge of the formula $-SiR^7{}_2-$, wherein both $R^7$ are the same $C_1$-$C_4$-hydrocarbyl or $C_6$-aryl group,
$R^1$ and $R^{1'}$ are the same and are a linear or branched $C_1$-$C_4$-alkyl group,
n is 1 or 2,
$R^2$ and $R^{2'}$ are the same and are a $CH_2$-$R^8$ group, with $R^8$ being H or linear or branched $C_1$-$C_3$-alkyl group
one of $R^5$ and $R^6$ or $R^{5'}$ and $R^{6'}$ form together an unsubstituted 5-6 membered ring condensed to the benzene ring of the indenyl moiety,
and the remaining residues of $R^5$ and $R^6$ or $R^{5'}$ and $R^6$, are for $R^5$ or $R^{5'}$ a OR group, wherein R is a $C_1$-$C_4$-alkyl group and for $R^6$ or $R^{6'}$ a $C(R^9)_3$ group, with $R^9$ being the same and $R^9$ can be a linear or branched $C_1$-$C_2$-alkyl group, which are suitable for being used in the present invention.

Viewed from yet another aspect the invention provides a process for the preparation of an ethylene copolymer comprising polymerizing ethylene and a $C_{4-10}$ alpha-olefin comonomer in a high temperature solution process at a temperature greater than 100°C in the presence of a catalyst comprising:

(i) a metallocene complex of formula (I) as defined above
(ii) an aluminoxane cocatalyst and
(iii) optionally a boron containing cocatalyst.

Viewed from a further aspect the invention provides an ethylene copolymer made by a process as hereinbefore defined.

## Detailed Description of the Invention

### Metallocene Complex

The single site metallocene complex, especially the complexes defined by the formula (I) specified in the present invention, used for manufacture of the ethylene copolymer are symmetrical or asymmetrical. For asymmetrical complexes that means that the two indenyl ligands forming the metallocene complex are different, that is, each indenyl ligand bears a set of substituents that are either chemically different, or located in different positions with respect to the other indenyl ligand. More precisely, they are chiral, racemic bridged bisindenyl metallocene complexes.

Whilst the complexes of the invention may be in their syn configuration, ideally they are in their anti configuration. For the purpose of this invention, racemic-anti means that the two indenyl ligands are oriented in opposite directions with respect to the cyclopentadienyl-metal-cyclopentadienyl plane, while racemic-syn means that the two indenyl ligands are oriented in the same direction with respect to the cyclopentadienyl-metal-cyclopentadienyl plane, as shown in the Figure below.

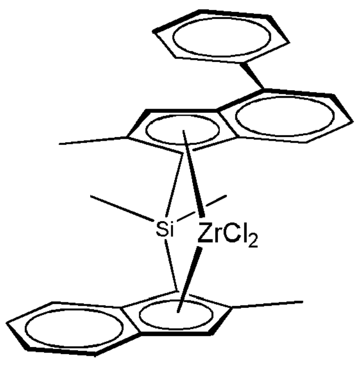

Racemic Anti

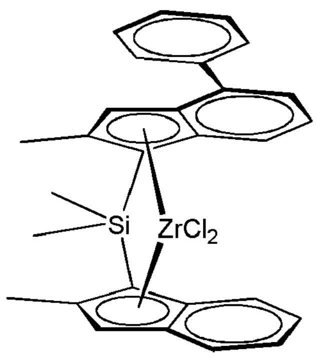

Racemic Syn

Formula (I) is intended to cover both syn and anti configurations.

By nature of their chemistry, both anti and syn enantiomer pairs are formed during the synthesis of the complexes. However, by using the ligands of this invention, separation of the preferred anti isomers from the syn isomers is straightforward.

It is preferred if the metallocene complexes of the invention are employed as the rac anti isomer. Ideally therefore at least 95% mol, such as at least 98% mol, especially at least 99% mol of the metallocene catalyst is in the racemic anti isomeric form.

The invention can be effected with a metallocene complex of formula (I)

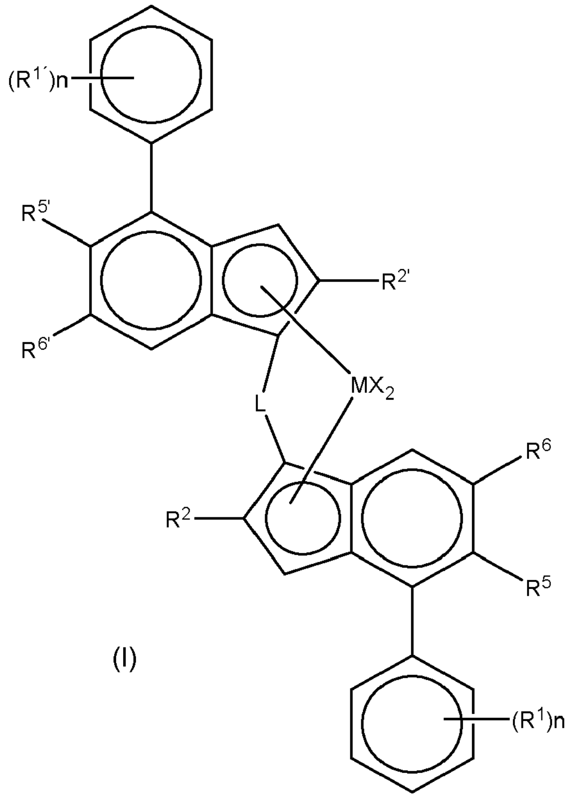

(I)

wherein

- M is Hf or Zr, preferably Zr
- X is a sigma ligand
- L is a bridge of the formula $-SiR^7_2-$, wherein each $R^7$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl
- $R^1$ and $R^{1'}$ are the same or can be different and can be a linear or branched $C_1$-$C_6$-alkyl group
- n is 0, 1 or 2
- $R^2$ and $R^{2'}$ are the same or can be different and are a $CH_2$-$R^8$ group, with $R^8$ being H or linear or branched $C_1$-$C_6$-alkyl group
- $R^5$ and $R^{5'}$ are the same or are different and can be a linear or branched $C_1$-$C_6$-alkyl group or a OR group, wherein R is a $C_1$-$C_6$-alkyl group
- $R^6$ and $R^{6'}$ are the same or are different and can be a $C(R^9)_3$ group, with $R^9$ being the same or different and $R^9$ can be H or a linear or branched $C_1$-$C_6$-alkyl group
- or $R^5$ and $R^6$ and/or $R^{5'}$ and $R^{6'}$ taken together form an unsubstituted 4-7 membered ring condensed to the benzene ring of the indenyl moiety,

with the proviso that if $R^5$ and $R^6$ as well as $R^{5'}$ and $R^{6'}$ taken together form an unsubstituted 5 membered ring condensed to the benzene ring of the indenyl moiety, then $R^2$ and $R^{2'}$ are not a $C_1$-alkyl group.

In the formula (I) each X, which may be the same or different, is a sigma ligand, preferably a hydrogen atom, a halogen atom, a $R^{10}$, $OR^{10}$, $OSO_2CF_3$, $OCOR^{10}$, $SR^{10}$, $NR^{10}_2$ or $PR^{10}_2$ group wherein $R^{10}$ is a linear or branched, cyclic or acyclic, $C_1$-$C_{20}$-alkyl, $C_2$-$C_{20}$-alkenyl, $C_2$-$C_{20}$-alkynyl, $C_6$-$C_{20}$-aryl, $C_7$-$C_{20}$-alkylaryl or $C_7$-$C_{20}$-arylalkyl radical; optionally containing heteroatoms belonging to groups 14-16 or is $SiR^{10}_3$, $SiHR^{10}_2$ or $SiH_2R^{10}$. $R^{10}$ is preferably a $C_{1-6}$-alkyl, phenyl or benzyl group, whereby the term halogen includes fluoro, chloro, bromo and iodo groups, preferably chloro groups.

More preferably each X is independently a hydrogen atom, a halogen atom, $C_{1-6}$-alkoxy group or an $R^{10}$ group, e.g. preferably a $C_{1-6}$-alkyl, phenyl or benzyl group.

Most preferably X is chlorine or a methyl radical. Preferably both X groups are the same.

$R^1$ and $R^{1'}$ are the same and are a linear or branched $C_1$-$C_6$-alkyl group, preferably a linear or branched $C_2$-$C_6$-alkyl group, more preferably a linear or branched butyl-group and most preferably $R^1$ and $R^{1'}$ are tert.-butyl.

n is 0, 1 or 2, preferably 1 or 2,

If n is 1, then $R^1$ and $R^{1'}$ are preferably on position 4 (para) of the phenyl ring and if n is 2 then $R^1$ and $R^{1'}$ are preferably on positions 3 and 5 of the phenyl ring.

$R^2$ and $R^{2'}$are the same or can be different and are a $CH_2$-$R^8$ group, with $R^8$ being H or linear or branched $C_1$-$C_6$-alkyl group, like methyl, ethyl, n-propyl, i-propyl, n-butyl, i-butyl, sec.-butyl and tert.-butyl. Preferably $R^2$ and $R^{2'}$ are the same and are a $CH_2$-$R^8$ group, with $R^8$ being H or linear or branched $C_1$-$C_4$-alkyl group, more preferably $R^2$ and $R^{2'}$ are the same and are a $CH_2$-$R^8$ group, with $R^8$ being H or linear or branched $C_1$-$C_3$-alkyl group and most preferably $R^2$ and $R^{2'}$ are either both methyl or both i-butyl.

$R^5$ and $R^{5'}$ are the same or are different and can be a linear or branched $C_1$-$C_6$-alkyl group or a OR group, wherein R is a linear or branched $C_1$-$C_6$-alkyl group, and $R^6$ and $R^{6'}$ are the same or are different and can be a $C(R^9)_3$ group, with $R^9$ being the same or different and $R^9$ can be H or a linear or branched $C_1$-$C_6$-alkyl group,

or $R^5$ and $R^6$ and/or $R^{5'}$ and $R^{6'}$ taken together form an unsubstituted 4-7, preferably 5-6 membered ring condensed to the benzene ring of the indenyl moiety.

Preferably at least one of $R^5$ and $R^6$ or $R^5$ and $R^{6'}$ together form an unsubstituted 4-7, preferably 5-6 membered ring condensed to the benzene ring of the indenyl moiety. More preferably at least one of $R^5$ and $R^6$ or $R^{5'}$ and $R^{6'}$ form an unsubstituted 5 membered ring condensed to the benzene ring of the indenyl moiety.

If only one of $R^5$ and $R^6$ or $R^{5'}$ and $R^{6'}$ together form an unsubstituted 4-7, preferably 5-6 membered ring condensed to the benzene ring of the indenyl moiety, then the substituents on the other indenyl moiety (either $R^5$ and $R^6$ or $R^{5'}$ and $R^{6'}$) are preferably (for $R^5$ or $R^{5'}$) a OR group wherein R is a linear or branched $C_1$-$C_6$-alkyl group, like methyl, ethyl, n-propyl, i-propyl, n-butyl, i-butyl, sec.-butyl and tert.-butyl, preferably a linear $C_1$-$C_4$-alkyl group, more preferably a $C_1$-$C_2$-alkyl group and most preferably a $C_1$-alkyl group and (for $R^6$ or $R^{6'}$) a $C(R^9)_3$ group, with $R^9$ being the same or different and $R^9$ can be H or a linear or branched $C_1$-$C_6$-alkyl group, preferably with $R^9$ being the same or different and $R^9$ being a linear or branched $C_1$-$C_4$-alkyl group, more preferably with $R^9$ being the same and $R^9$ being a $C_1$-$C_2$-alkyl group, most preferably the $C(R^9)_3$ group is a tert.-butyl group.

L is a bridge of the formula -$SiR^7_2$-, wherein each $R^7$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl.

The term $C_{1-20}$ hydrocarbyl group therefore includes $C_{1-20}$ alkyl, $C_{2-20}$ alkenyl, $C_{2-20}$ alkynyl, $C_{3-20}$ cycloalkyl, $C_{3-20}$ cycloalkenyl, $C_{6-20}$ aryl groups, $C_{7-20}$ alkylaryl groups or $C_{7-20}$ arylalkyl groups or of course mixtures of these groups such as cycloalkyl substituted by alkyl.

Unless otherwise stated, preferred $C_{1-20}$ hydrocarbyl groups are $C_{1-20}$ alkyl, $C_{4-20}$ cycloalkyl, $C_{5-20}$ cycloalkyl-alkyl groups, $C_{7-20}$ alkylaryl groups, $C_{7-20}$ arylalkyl groups or $C_{6-20}$ aryl groups.

Preferably $R^7$ are the same and are a $C_1$-$C_{10}$-hydrocarbyl or $C_6$-$C_{10}$-aryl group, like methyl, ethyl, propyl, isopropyl, tertbutyl, isobutyl, $C_{5-6}$-cycloalkyl, cyclohexylmethyl, phenyl or benzyl, more preferably both $R^7$ are a $C_1$-$C_4$-hydrocarbyl or $C_6$-aryl group and most preferably both $R^7$ are a $C_1$-alkyl group.

Especially preferred complexes of formula (I) are

*racemic* dimethylsilanediylbis[2-*iso*-butyl-4-(4-*tert*-butylphenyl)-5,6,7-trihydro-s-indacen-1-yl] zirconium dichloride or dimethyl and racemic dimethylsilanediyl[η5-6-*tert*-butyl-4-(3,5-di-*tert*-butylphenyl)-5-methoxy-2-methylinden-1-yl][η5-4-(3,5-di-*tert*-butylphenyl)-2-methyl-5,6,7-trihydro-s-indacen-1-yl] zirconium dichloride or dimethyl,

either in their syn or anti configuration.

For the purpose of this invention, the terms dimethylsilyl, dimethylsilanediyl and dimethylsililene are equivalent.

The metallocenes of formula (I) as described above include a new class of metallocenes, which are suitable for being used in the present invention, wherein in the formula (I)

M is Zr,

X is Cl or a methyl group

L is a bridge of the formula -$SiR^7_2$-, wherein both $R^7$ are the same $C_1$-$C_4$-hydrocarbyl or $C_6$-aryl group,

$R^1$ and $R^{1'}$ are the same and are a linear or branched $C_1$-$C_4$-alkyl group,

n is 1 or 2,

$R^2$ and $R^{2'}$are the same and are a $CH_2$-$R^8$ group, with $R^8$ being H or a $C_1$-$C_3$-alkyl group one of $R^5$ and $R^6$ or $R^{5'}$ and $R^{6'}$ form together an unsubstituted 5-6 membered ring condensed to the benzene ring of the indenyl moiety,

and the remaining residues of $R^5$ and $R^6$ or $R^{5'}$ and $R^6$, are for $R^5$ or $R^{5'}$ a OR group, wherein R is a $C_1$-$C_4$-alkyl group and for $R^6$ or $R^{6'}$ a $C(R^9)_3$ group, with $R^9$ being the same and $R^9$ can be a $C_1$-$C_2$-alkyl group.

This new class of metallocenes is a further embodiment of the invention.

**Synthesis**

The ligands required to form the catalysts of the invention can be synthesised by any process and the skilled organic chemist would be able to devise various synthetic protocols for the manufacture of the necessary ligand materials. WO2007/116034 discloses the necessary chemistry and is herein incorporated by reference. Synthetic protocols can also generally be found in WO2002/02576, WO2011/135004, WO2012/084961, WO2012/001052 and WO2011/076780.

**Cocatalyst**

To form an active catalytic species it is normally necessary to employ a cocatalyst as is well known in the art. The present invention requires the use of an aluminoxane cocatalyst and optionally an additional boron containing cocatalyst.
The aluminoxane cocatalyst can be one of formula (II):

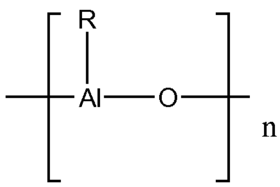
(II)

where n is usually from 6 to 20 and R has the meaning below.
Aluminoxanes are formed on partial hydrolysis of organoaluminum compounds, for example those of the formula $AlR_3$, $AlR_2Y$ and $Al_2R_3Y_3$ where R can be, for example, C1-C10 alkyl, preferably C1-C5 alkyl, or C3-10-cycloalkyl, C7-C12 -arylalkyl or alkylaryl and/or phenyl or naphthyl, and where Y can be hydrogen, halogen, preferably chlorine or bromine, or C1-C10 alkoxy, preferably methoxy or ethoxy. The resulting oxygen-containing aluminoxanes are not in general pure compounds but mixtures of oligomers of the formula (I).
The preferred aluminoxane in the process according to the invention is methylaluminoxane (MAO). Since the aluminoxanes used according to the invention as cocatalysts are not, owing to their mode of preparation, pure compounds, the molarity of aluminoxane solutions hereinafter is based on their aluminium content.
Boron based cocatalysts of interest include boron compounds containing a borate $3^+$ ion, i.e. borate compounds. These compounds generally contain an anion of formula:

$(Z)_4B^-$ (III)

where Z is an optionally substituted phenyl derivative, said substituent being a halo$C_{1-6}$-alkyl or halo group. Preferred options are fluoro or trifluoromethyl. Most preferably, the phenyl group is perfluorinated.
Such ionic cocatalysts preferably contain a non-coordinating anion such as tetrakis(pentafluorophenyl)borate.
Suitable counterions are protonated amine or aniline derivatives or phosphonium ions. These may have the general formula (IV) or (V):

$NQ_4^+$ (IV) or $PQ_4^+$ (V)

where Q is independently H, $C_{1-6}$-alkyl, $C_{3-8}$ cycloalkyl, phenyl$C_{1-6}$-alkylene- or optionally substituted Ph. Optional substituents may be $C_{1-6}$-alkyl, halo or nitro. There may be one or more than one such substituent. Preferred substituted Ph groups include therefore parasubstituted phenyl, preferably tolyl or dimethylphenyl.
It is preferred if at least one Q group is H, thus preferred compounds are those of formula:

$NHQ_3^+$ (VI) or $PHQ_3^+$ (VII)

Preferred phenyl$C_{1-6}$-alkyl- groups include benzyl.
Suitable counterions therefore include: methylammonium, anilinium, dimethylammonium, diethylammonium, N-methylanilinium, diphenylammonium, N,N-dimethylanilinium, trimethylammonium, triethylammonium, tri-n-butylammonium, methyldiphenylammonium, p-bromo-N,N- dimethylanilinium or p-nitro-N,N-dimethylanilinium, especially dimethylammonium or N,N-dimethylanilinium. The use of pyridinium as an ion is a further option.

Phosphonium ions of interest include triphenylphosphonium, triethylphosphonium, diphenylphosphonium, tri(methylphenyl)phosphonium and tri(dimethylphenyl)phosphonium. A more preferred counterion is trityl ($CPh_3^+$) or analogues thereof in which the Ph group is functionalised to carry one or more alkyl groups. Highly preferred borates of use in the invention therefore comprise the tetrakis(pentafluorophenyl)borate ion.
Preferred ionic compounds which can be used according to the present invention include:

tributylammoniumtetra(pentafluorophenyl)borate,
tributylammoniumtetra(trifluoromethylphenyl)borate,
tributylammoniumtetra-(4-fluorophenyl)borate,
N,N-dimethylcyclohexylammoniumtetrakis-(pentafluorophenyl)borate,
N,N-dimethylbenzylammoniumtetrakis(pentafluorophenyl)borate,
N,N-dimethylaniliniumtetrakis(pentafluorophenyl)borate,
N,N- di(propyl)ammoniumtetrakis(pentafluorophenyl)borate,
di(cyclohexyl)ammoniumtetrakis(pentafluorophenyl)borate,
triphenylcarbeniumtetrakis(pentafluorophenyl)borate,
ferroceniumtetrakis(pentafluorophenyl)borate.

Preference is given to triphenylcarbeniumtetrakis(pentafluorophenyl) borate,

N,N- dimethylcyclohexylammoniumtetrakis(pentafluorophenyl)borate,
N,N- dimethylbenzylammoniumtetrakis(pentafluorophenyl)borate or
N,N-dimethylaniliniumtetrakis(pentafluorophenyl)borate.

It has been surprisingly found that certain boron cocatalysts are especially preferred. Preferred borates of use in the invention therefore comprise the trityl ion. Thus the use of N,N-dimethylammonium-tetrakispentafluorophenylborate and $Ph_3CB(PhF_5)_4$ and analogues therefore are especially favoured.
Preferably both cocatalysts, an aluminoxane and a boron based cocatalyst, are used in the catalyst system of the present invention.
It is further possible to add an aluminium alkyl compound. Suitable aluminium alkyl compounds are compounds of the formula (VIII) $AlR_3$ with R being a linear or branched $C_2$-$C_8$-alkyl group.
Preferred aluminium alkyl compounds are triethylaluminium, tri-isobutylaluminium, triisohexylaluminium, tri-n-octylaluminium and tri-isooctylaluminium.
Suitable amounts of cocatalyst will be well known to the skilled man.
The molar ratio of boron to the metal ion of the metallocene may be in the range 0.5:1 to 10:1 mol/mol, preferably 1:1 to 10:1, especially 1:1 to 5:1 mol/mol.
The molar ratio of Al in the aluminoxane to the metal ion of the metallocene may be in the range 1:1 to 2000:1 mol/mol, preferably 10:1 to 1000:1, more preferably 50:1 to 500:1 mol/mol.

**Catalyst Manufacture**

The metallocene complex of the present invention is used in combination with the cocatalyst(s) as a catalyst system for the polymerization of ethylene and $C_{4-10}$ alpha-olefin comonomer in a high temperature solution polymerization process.
The catalyst system of the invention can be used in non-supported form or in solid form. The catalyst system of the invention may be used as a homogeneous catalyst or heterogeneous catalyst.
The catalyst system of the invention in solid form, preferably in solid particulate form is free from an external carrier, however still being in solid form.
By free from an external carrier is meant that the catalyst does not contain an external support, such as an inorganic support, for example, silica or alumina, or an organic polymeric support material.

**a) Non-supported**

Non-supported catalyst systems, suitable for the present invention can be prepared in solution, for example in an aromatic solvent like toluene, by contacting the metallocene (as a solid or as a solution) with the cocatalyst(s), for example methylaluminoxane and/or a borane or a borate salt previously dissolved in an aromatic solvent, or can be prepared by sequentially adding the catalyst components to the polymerization medium.

**b) Solid form**

In an alternative embodiment, in order to provide the catalyst system of the invention in solid form but without using an external carrier, it is preferred if a liquid/liquid emulsion system is used. The process involves forming dispersing catalyst components (i) (the complex) and (ii) + optionally (iii) the cocatalysts) in a solvent, and solidifying said dispersed droplets to form solid particles.

In the present case, if, aluminoxane as well as boron based cocatalysts are used, it is particularly preferred if the aluminoxane is contacted with the metallocene before the borate is added. Both cocatalyst components and the metallocene are preferably present in one solution.

In particular, the method involves preparing a solution of the catalyst components; dispersing said solution in an solvent to form an emulsion in which said one or more catalyst components are present in the droplets of the dispersed phase; immobilising the catalyst components in the dispersed droplets, in the absence of an external particulate porous support, to form solid particles comprising the said catalyst, and optionally recovering said particles.

This process enables the manufacture of active catalyst particles with improved morphology, e.g. with a predetermined particle size, spherical shape, compact structure, excellent surface properties and without using any added external porous support material, such as an inorganic oxide, e.g. silica. The catalyst particles can have a smooth surface, they may be compact in nature and catalyst active components can be distributed uniformly thorough the catalyst particles.

Full disclosure of the necessary process steps can be found in WO03/051934 which is herein incorporated by reference.

All or part of the preparation steps can be done in a continuous manner. Reference is made to WO2006/069733 describing principles of such a continuous or semicontinuous preparation methods of the solid catalyst types, prepared via emulsion/solidification method.

The formed catalyst preferably has good stability/kinetics in terms of longevity of reaction, high activity and the catalysts enable low ash contents.

The use of the heterogeneous, non-supported catalysts, (i.e. "self-supported" catalysts) might have, as a drawback, a tendency to dissolve to some extent in the polymerization media, i.e. some active catalyst components might leach out of the catalyst particles during slurry polymerization, whereby the original good morphology of the catalyst might be lost. These leached catalyst components are very active possibly causing problems during polymerization. Therefore, the amount of leached components should be minimized, i.e. all catalyst components should be kept in heterogeneous form.

Furthermore, the self-supported catalysts generate, due to the high amount of catalytically active species in the catalyst system, high temperatures at the beginning of the polymerization which may cause melting of the product material. Both effects, i.e. the partial dissolving of the catalyst system and the heat generation, might cause fouling, sheeting and deterioration of the polymer material morphology.

In order to minimise the possible problems associated with high activity or leaching, it is preferred to "prepolymerize" the catalyst before using it in polymerization process. It has to be noted that prepolymerization in this regard is part of the catalyst preparation process, being a step carried out after a solid catalyst is formed. This catalyst prepolymerization step is not part of the actual polymerization configuration, which might comprise a conventional process prepolymerization step as well. After the catalyst prepolymerization step, a solid catalyst is obtained and used in polymerization.

Catalyst "prepolymerization" takes place following the solidification step of the liquid-liquid emulsion process hereinbefore described. Prepolymerization may take place by known methods described in the art, such as that described in WO 2010/052263, WO 2010/052260 or WO 2010/052264. Preferable embodiments of this aspect of the invention are described herein.

Use of the catalyst prepolymerization step offers the advantage of minimising leaching of catalyst components and thus local overheating.

**Polymer**

The polymer to be produced using the catalyst system of the invention is copolymer of ethylene and a $C_{4-10}$ alpha-olefin comonomer, like 1-butene, 1-hexene, 4-methyl-1-pentene, 1-octene etc. Preferably butene, hexene or octene and more preferably octene is used as comonomer.

The comonomer content in such a polymer may be up to 40 wt%, preferably between 5 to 40 wt%, more preferably 10 to 38 wt% and more preferable 15 to 36 wt%.

The density (measured according to ISO 1183-187) of the polymers is in the range of 0.850 g/cm$^3$ to 0.950 g/cm$^3$, preferably in the range of 0.850 g/cm$^3$ to 0.945 g/cm$^3$ and more preferably in the range of 0.850 g/cm$^3$ to 0.940 g/cm$^3$.

Mw/Mn value of the polymers of the invention is less than 5, e.g. in the range of 2.0 to 4.5.

The melting points (measured with DSC according to ISO 11357-3:1999) of the polymers to be produced are below 130°C, preferably below 120°C, more preferably below 110°C and most preferably below 100°C

**Polymerization**

The catalyst system of the present invention is used to produce the above defined ethylene copolymers in a high temperature solution polymerization process at temperatures higher than 100°C.

In view of this invention such process is essentially based on polymerizing the monomer and a suitable comonomer in a liquid hydrocarbon solvent in which the resulting polymer is soluble. The polymerization is carried out at a temperature above the melting point of the polymer, as a result of which a polymer solution is obtained. This solution is flashed in order to separate the polymer from the unreacted monomer and the solvent. The solvent is then recovered and recycled in the process.

A solution polymerization process is known for its short reactor residence times (compared to Gas-phase or slurry processes) allowing, thus, very fast grade transitions and significant flexibility in producing a wide product range in a short production cycle.

According to the present invention the used solution polymerization process is a high temperature solution polymerization process, using a polymerization temperature of higher than 100°C. Preferably the polymerization temperature is at least 110°, more preferably at least 150°C. The polymerization temperature can be up to 250°C.

The pressure in the used solution polymerization process according to the invention is preferably in a range of 10 to 100 bar, preferably 15 to 100 bar and more preferably 20 to 100 bar.

The liquid hydrocarbon solvent used is preferably a $C_{5-12}$-hydrocarbon which may be unsubstituted or substituted by $C_{1-4}$ alkyl group such as pentane, methyl pentane, hexane, heptane, octane, cyclohexane, methylcyclohexane and hydrogenated naphtha. More preferably unsubstituted $C_{6-10}$-hydrocarbon solvents are used.

A known solution technology suitable for the process according to the invention is the COMPACT technology.

**Advantage**

The new catalyst systems, comprising component (i), (ii) and optionally (iii) can be advantageously used for ethylene copolymerization in high temperature solution polymerization process.

The catalyst systems according to the present invention show excellent productivity, excellent comonomer incorporation and thermal stability if used for ethylene copolymerization in high temperature solution polymerization process.

**Applications**

The polymers made by the catalyst system of the invention are useful in all kinds of end articles such as pipes, films (cast or blown films), fibers, moulded articles (e.g. injection moulded, blow moulded, rotomoulded articles), extrusion coatings and so on.

The invention will now be illustrated by reference to the following non-limiting examples

**Examples:**

**Methods**

**Al and Zr determination (ICP-method)**

The elemental analysis of a catalyst was performed by taking a solid sample of mass, m. The catalyst was deactivated by substituting the inert storing conditions with ambient air, first passively through a needle and the actively by applying vacuum three times to the sampling container. Samples were dissolved to a volume V by first cooling on dry ice while adding freshly deionised water (5% of V) and nitric acid ($HNO_3$, 65 %, 5 % of V). The samples were transferred in full to volumetric flasks using deionised water and rinsing the sampling containers. Hydrofluoric acid (HF, 40 %, 3 % of V) was added to the volumetric flasks and volume V obtained by addition of freshly deionised water. The prepared sample solutions were left to stabilise for two hours.

The analysis was run at room temperature using a Thermo Elemental iCAP 6300 Inductively Coupled Plasma - Optical Emission Spectrometer (ICP-OES) which was calibrated using a blank (a solution of 5 % HNO3, 5 % HF in deionised water), and 6 standards of 0.5 ppm, 1 ppm, 10 ppm, 50 ppm, 100 ppm and 300 ppm of Al, with 0.5 ppm, 1 ppm, 5 ppm, 20 ppm, 50 ppm and 100 ppm of B and P in solutions of 5 % HNO3, 3 % HF in deionised water. Immediately before analysis the calibration is 'resloped' using the blank and 100 ppm Al, 50 ppm B, P standard, a quality control sample

(20 ppm Al, 5 ppm B, P in a solution of 5 % HNO3, 3 % HF in DI water) is run to confirm the reslope. The QC sample is also run after every 5th sample and at the end of a scheduled analysis set.

The reported values are an average of three successive aliquots taken from the same sample and are related back to the original catalyst by inputting the original mass of sample, m, and the dilution volume, V, into the software.

**DSC analysis**

The melting point ($T_m$) and crystallization temperature ($T_c$) were determined on a DSC200 TA instrument, by placing a 5-7 mg polymer sample, into a closed DSC aluminum pan, heating the sample from -30 °C to 180 °C at 10 °C/min, holding for 5 min at 180 °C, cooling from 180 °C to -30 °C, holding for 5 min at -30 °C, heating from -30 °C to 180 °C at 10 °C/min. The reported $T_m$ is the maximum of the curve from the second heating scan and $T_c$ is the maximum of the curve of the cooling scan.

**Quantification of comonomer content by NMR spectroscopy**

Quantitative nuclear-magnetic resonance (NMR) spectroscopy was used to quantify the comonomer content of the polymers.

Quantitative $^{13}C\{^1H\}$ NMR spectra recorded in the molten-state using a Bruker Advance III 500 NMR spectrometer operating at 500.13 and 125.76 MHz for $^1H$ and $^{13}C$ respectively. All spectra were recorded using a $^{13}C$ optimised 7 mm magic-angle spinning (MAS) probehead at 150°C using nitrogen gas for all pneumatics. Approximately 200 mg of material was packed into a 7 mm outer diameter zirconia MAS rotor and spun at 4 kHz. This setup was chosen primarily for the high sensitivity needed for rapid identification and accurate quantification.{klimke06, parkinson07, castignolles09, parkinson11} Standard single-pulse excitation was employed utilising the transient NOE at short recycle delays of 3s {pollard04, klimke06} and the RS-HEPT decoupling scheme{fillip05,griffin07}. A total of 1024 (1k) transients were acquired per spectrum. This setup was chosen due its high sensitivity towards low comonomer contents.

Quantitative $^{13}C\{^1H\}$ NMR spectra were processed, integrated and quantitative properties determined using custom spectral analysis automation programs. All chemical shifts are internally referenced to the bulk methylene signal (δ+) at 30.00 ppm {randall89}.

Characteristic signals corresponding to the incorporation of 1-octene were observed (randall89, liu01, qiu07) and all comonomer contents calculated with respect to all other monomers present in the polymer.

Characteristic signals resulting from isolated 1-octene incorporation i.e. EEOEE comonomer sequences, were observed. Isolated 1-octene incorporation was quantified using the integral of the signal at 38.32 ppm. This integral is assigned to the unresolved signals corresponding to both *B6 and *βB6B6 sites of isolated (EEOEE) and isolated double non-consecutive (EEOEOEE) 1-octene sequences respectively. To compensate for the influence of the two *βB6B6 sites the integral of the ββB6B6 site at 24.7 ppm is used:

$$O = I_{*B6+*\beta B6B6} - 2 * I_{\beta\beta B6B6}$$

Characteristic signals resulting from consecutive 1-octene incorporation, i.e. EEOOEE comonomer sequences, were also observed. Such consecutive 1-octene incorporation was quantified using the integral of the signal at 40.48 ppm assigned to the ααB6B6 sites accounting for the number of reporting sites per comonomer:

$$OO = 2 * I_{\alpha\alpha B6B6}$$

Characteristic signals resulting from isolated non-consecutive 1-octene incorporation, i.e. EEOEOEE comonomer sequences, were also observed. Such isolated non-consecutive 1-octene incorporation was quantified using the integral of the signal at 24.7 ppm assigned to the ββB6B6 sites accounting for the number of reporting sites per comonomer:

$$OEO = 2 * I_{\beta\beta B6B6}$$

Characteristic signals resulting from isolated triple-consecutive 1-octene incorporation, i.e. EEOOOEE comonomer sequences, were also observed. Such isolated triple-consecutive 1-octene incorporation was quantified using the integral of the signal at 41.2 ppm assigned to the ααγB6B6B6 sites accounting for the number of reporting sites per comonomer:

$$OOO = 3/2 * I_{\alpha\alpha\gamma B6B6B6}$$

With no other signals indicative of other comonomer sequences observed the total 1-octene comonomer content was calculated based solely on the amount of isolated (EEOEE), isolated double-consecutive (EEOOEE), isolated non-consecutive (EEOEOEE) and isolated triple-consecutive (EEOOOEE) 1-octene comonomer sequences:

$$O_{total} = O + OO + OEO + OOO$$

Characteristic signals resulting from saturated end-groups were observed. Such saturated end-groups were quantified using the average integral of the two resolved signals at 22.84 and 32.23 ppm. The 22.84 ppm integral is assigned to the unresolved signals corresponding to both 2B6 and 2S sites of 1-octene and the saturated chain end respectively. The 32.23 ppm integral is assigned to the unresolved signals corresponding to both 3B6 and 3S sites of 1-octene and the saturated chain end respectively. To compensate for the influence of the 2B6 and 3B6 1-octene sites the total 1-octene content is used:

$$S = (1/2)*( I_{2S+2B6} + I_{3S+3B6} - 2*O_{total})$$

The ethylene comonomer content was quantified using the integral of the bulk methylene (bulk) signals at 30.00 ppm. This integral included the $\gamma$ and 4B6 sites from 1-octene as well as the $\delta^+$ sites. The total ethylene comonomer content was calculated based on the bulk integral and compensating for the observed 1-octene sequences and end-groups:

$$E_{total} = (1/2)*[ I_{bulk} + 2*O + 1*OO + 3*OEO + 0*OOO + 3*S ]$$

It should be noted that compensation of the bulk integral for the presence of isolated triple-incorporation (EEOOOEE) 1-octene sequences is not required as the number of under and over accounted ethylene units is equal.
The total mole fraction of 1-octene in the polymer was then calculated as:

$$fO = ( O_{total} / ( E_{total} + O_{total} )$$

The total comonomer incorporation of 1-octene in weight percent was calculated from the mole fraction in the standard manner:

$$O\ [wt\%] = 100 * ( fO * 112.21) / ( (fO * 112.21) + ((1-fO) * 28.05) )$$

klimke06
Klimke, K., Parkinson, M., Piel, C., Kaminsky, W., Spiess, H.W., Wilhelm, M., Macromol. Chem. Phys. 2006;207:382.

parkinson07
Parkinson, M., Klimke, K., Spiess, H.W., Wilhelm, M., Macromol. Chem. Phys. 2007;208:2128.

parkinson 11
NMR Spectroscopy of Polymers: Innovative Strategies for Complex Macromolecules, Chapter 24, 401 (2011)

pollard04
Pollard, M., Klimke, K., Graf, R., Spiess, H.W., Wilhelm, M., Sperber, O., Piel, C., Kaminsky, W., Macromolecules 2004;37:813.

filip05
Filip, X., Tripon, C., Filip, C., J. Mag. Resn. 2005, 176, 239

griffin07

Griffin, J.M., Tripon, C., Samoson, A., Filip, C., and Brown, S.P., Mag. Res. in Chem. 2007 45, S1, S198

castignolles09
Castignolles, P., Graf, R., Parkinson, M., Wilhelm, M., Gaborieau, M., Polymer 50 (2009) 2373

zhou07
Zhou, Z., Kuemmerle, R., Qiu, X., Redwine, D., Cong, R., Taha, A., Baugh, D. Winniford, B., J. Mag. Reson. 187 (2007) 225

busico07
Busico, V., Carbonniere, P., Cipullo, R., Pellecchia, R., Severn, J., Talarico, G., Macromol. Rapid Commun. 2007, 28, 1128

randall89
J. Randall, Macromol. Sci., Rev. Macromol. Chem. Phys. 1989, C29, 201.

qui07
Qiu, X., Redwine, D., Gobbi, G., Nuamthanom, A., Rinaldi, P., Macromolecules 2007, 40, 6879

liu01
Liu, W., Rinaldi, P., McIntosh, L., Quirk, P., Macromolecules 2001, 34, 4757

GPC: Molecular weight averages, molecular weight distribution, and polydispersity index ($M_n$, $M_w$, $M_w/M_n$)

Molecular weight averages (Mw, Mn), Molecular weight distribution (MWD) and its broadness, described by polydispersity index, PDI= Mw/Mn (wherein Mn is the number average molecular weight and Mw is the weight average molecular weight) were determined by Gel Permeation Chromatography (GPC) according to ISO 16014-4:2003 and ASTM D 6474-99. A Waters GPCV2000 instrument, equipped with differential refractive index detector and online viscosimeter was used with 2 x GMHXL-HT and 1x G7000HXL-HT TSK-gel columns from Tosoh Bioscience and 1,2,4-trichlorobenzene (TCB, stabilized with 250 mg/L 2,6-Di tert butyl-4-methyl-phenol) as solvent at 140 °C and at a constant flow rate of 1 mL/min. 209.5 μL of sample solution were injected per analysis. The column set was calibrated using universal calibration (according to ISO 16014-2:2003) with at least 15 narrow MWD polystyrene (PS) standards in the range of 1 kg/mol to 12 000 kg/mol. Mark Houwink constants for PS, PE and PP used are as per ASTM D 6474-99. All samples were prepared by dissolving 0.5 - 4.0 mg of polymer in 4 mL (at 140 °C) of stabilized TCB (same as mobile phase) and keeping for max. 3 hours at max. 160°C with continuous gentle shaking prior sampling into the GPC instrument.

**Chemicals**

MAO was purchased from Chemtura and used as a 30 wt-% solution in toluene.

Triphenylcarbeniumtetrakis(pentafluorophenyl)borate (alternative name trityl tetrakis-(pentafluorophenyl)borate) (CAS 136040-19-2) was purchased from Acros (tritylBF20)

1-octene as co-monomer (99%, Sigma Aldrich) was dried over molecular sieves and degassed with nitrogen before use.

Heptane and decane (99.9 %, Sigma Aldrich) were dried under molecular sieves and degassed with nitrogen before use.

**Catalyst Preparation Examples**

For the purpose of this invention, the terms dimethylsilyl, dimethylsilanediyl and dimethylsililene are equivalent.

**a) Complex preparation:**

**Complex 1:**

*racemic* dimethylsilanediylbis[2-*iso*-butyl-4-(4-*tert*-butylphenyl)-5,6,7-trihydro-s-indacen-1-yl] zirconium dichloride was prepared as described in the patent application WO2012/001051A1. (C-1)

**Complex 2:**

Racemic dimethylsilanediyl[η[5]-6-*tert*-butyl-4-(3,5-di-*tert*-butylphenyl)-5-methoxy-2-methylinden-1-yl][η[5]-4-(3,5-di-*tert*-butylphenyl)-2-methyl-5,6,7-trihydro-s-indacen-1-yl] zirconium dichloride (C-2) is prepared according to the following procedure:

*General procedure*

1-*tert*-Butyl-2-methoxybenzene was synthesized via alkylation of 2-*tert*-butylphenol (Acros) by dimethylsulfate (Merck) in the presence of aqueous NaOH (Reachim, Russia) as described in [Stork, G.; White, W. N. J. Am. Chem. Soc. 1956, 78, 4604.]. 2-methyl-4-bromo-5-methoxy-6-tertbutylindanone was prepared as described in WO2013007650. *Bis*(2,6-diisopropylphenyl)imidazolium chloride, i.e. IPr(HCl), and (IPr)$NiCl_2(PPh_3)$ were synthesized as described in [Hintermann, L. Beilstein J. Org. Chem. 2007, 3, 1.] and [Matsubara, K.; Ueno, K.; Shibata, Y. Organometallics 2006, 25, 3422.], respectively. Anisole (Acros), 3-methylanisole (Acros), *tert*-Butyltoluene (Aldrich), $P_4O_{10}$ (Reachim), $Pd(P^tBu_3)_2$ (Strem), 1.0 M $ZnCl_2$ in THF (Aldrich), 1.0 M 3,5-di-*tert*-butylphenylmagnesium bromide in THF (Aldrich), hexanes (Reachim, Russia), N-bromosuccinimide (Acros), dry ethanol (Merck), diethyl methylmalonate (Aldrich), methyl iodide (Acros), acetone (Reachim, Russia), tetraethylammonium iodide (Acros), 1-Bromo-4-*tert*-butylbenzene (Acros), CuCN (Merck), methanesulfonic acid (Aldrich), sodium tetraphenylborate (Aldrich), palladium acetate (Aldrich), copper cyanide (Merck), lithium aluminiumhydride (Aldrich), bromobenzene (Acros), 2.5 M $^n$BuLi in hexanes (Chemetall), $ZrCl_4(THF)_2$ (Aldrich), $NaBH_4$ (Aldrich), $Ni(OAc)_2$ (Aldrich), silica gel 60 (40-63 um, Merck), $AlCl_3$ (Merck), bromine (Merck), benzoyl peroxide (Aldrich), iodine (Merck), $NaHCO_3$ (Merck), $Na_2CO_3$ (Merck), $K_2CO_3$ (Merck), $Na_2SO_4$ (Merck), $Na_2SO_3$ (Merck), sodium metal (Merck), thionyl chloride (Merck), magnesium turnings (Acros), sodium acetate, trihydrate (Merck), tetraethylammonium iodide (Acros), triphenylphosphine (Acros), KOH (Merck), $Na_2SO_4$ (Akzo Nobel), TsOH (Aldrich), 12 M HCl (Reachim, Russia), methanol (Merck), anhydrous ethanol (Merck), $CDCl_3$ and DMSO-$d_6$ (Deutero GmbH) as well as hexanes (Merck), carbon tetrachloride (Merck), ether (Merck), ethyl acetate (Merck), toluene (Merck) and $CH_2Cl_2$ (Merck) for extractions were used as received. Tetrahydrofurane (Merck), ether (Merck), and dimethoxyethane (Acros) freshly distilled from benzophenone ketyl were used. Dichloromethane (Merck) for organometallic synthesis as well as $CD_2Cl_2$ (Deutero GmbH) for NMR experiments were dried and kept over $CaH_2$. Toluene (Merck), n-octane (Merck), and hexanes (Merck) for organometallic synthesis were kept and distilled over Na/K alloy. Dichlorodimethylsilane (Merck) and methacrylic acid (Acros) were distilled before use.

**6-*tert*-Butyl-4-(3,5-di-*tert*-butylphenyl)-5-methoxy-2-methylindan-1-one**

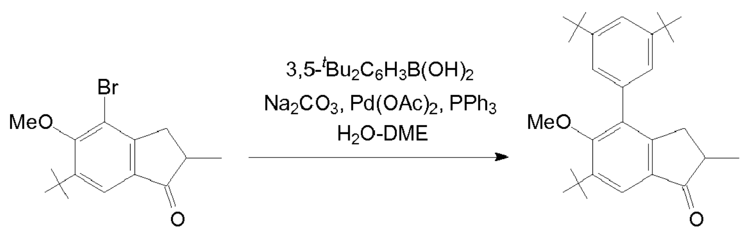


A mixture of 30.7g (98.6 mmol) of 2-methyl-4-bromo-5-methoxy-6-tertbutylindanone, 30.6 g (128 mmol) 3,5-di-*tert*-butylphenylboronic acid, 29.7 g (280 mmol) of $Na_2CO_3$, 1.35 g (5.92 mmol; 6 mol.%) of $Pd(OAc)_2$, 3.15 g (11.8 mmol; 12 mol.%) of $PPh_3$, 130 ml of water, and 380 ml of 1,2-dimethoxyethane was refluxed for 12 h. Further on, the reaction mixture was quenched with water, solvents were evaporated. The residue was dissolved in 500 ml of dichloromethane, and this solution was washed by 500 ml of water. The organic layer was separated, the aqueous layer was additionally extracted with 100 ml of dichloromethane. The combined organic extract was dried over $Na_2SO_4$, then evaporated to dryness. The crude product isolated from the residue using flash chromatography on silica gel 60 (40-63 um, hexanes-dichloromethane = 2:1, vol.) was then re-crystallized from n-hexane to give 18.5 g (43%) of a white solid. Anal. calc. for $C_{29}H_{40}O_2$: C, 82.81; H, 9.59. Found: C, 83.04; H, 9.75.
$^1$H NMR ($CDCl_3$): δ 7.74 (s, 1 H, 7-H in indan-1-one), 7.41 (t, J = 1.6 Hz, 1 H, 4-H in $C_6H_3{}^tBu_2$), 7.24 (d, J = 1.6 Hz, 2,6-H in $C_6H_3{}^tBu_2$), 3.24 (s, 3H, OMe), 3.17 (dd, J = 17.3 Hz, J = 8.0 Hz, 1 H, 3-H in indan-1-one), 2.64 (m, 1 H, 2-H in indan-1-one), 2.47 (dd, J = 17.3 Hz, J = 3.7 Hz, 1H, 3-H' in indan-1-one), 1.43 (s, 9H, 6-$^t$Bu in indan-1-one), 1.36 (s, 18H, $^t$Bu in $C_6H_3{}^tBu_2$), 1.25 (d, J = 7.3 Hz, 3H, 2-Me in indan-1-one).

**2-methyl-5-*tert*-Butyl-6-methoxy-7-(3,5-di-tert-butylphenyl)-1*H*-indene**

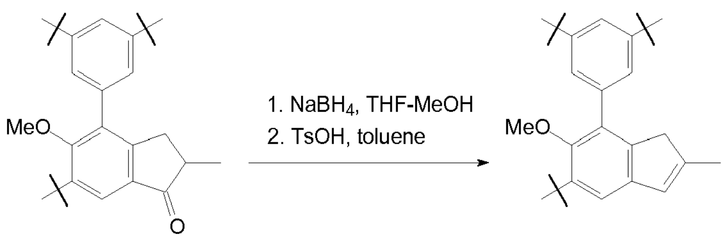


To a solution of 16.3 g (38.8 mmol) of 2-methyl-4-(3,5-di-*tert*-butylphenyl)-5-methoxy-6-*tert*-butyl-indan-1-one in 200 ml of THF cooled to 5°C 1.47 g (38.9 mmol) of $NaBH_4$ was added. Further on, 80 ml of methanol was added dropwise to this mixture by vigorous stirring for ca. 7 h at 5°C. The resulting mixture was evaporated to dryness, and the residue was treated by 300 ml of dichloromethane and 300 ml of 2 M HCl. The organic layer was separated, the aqueous layer was additionally extracted with 100 ml of dichloromethane. The combined organic extract was evaporated to dryness to give a colorless oil. To a solution of this oil in 250 ml of toluene 0.1 g of TsOH was added, this mixture was refluxed with Dean-Stark head for 15 min and then cooled to room temperature using water bath. The resulting solution was washed by 10% aqueous $Na_2CO_3$. The organic layer was separated, the aqueous layer was extracted with 2 x 50 ml of dichloromethane. The combined organic extract was dried over $K_2CO_3$ and then passed through a short layer of silica gel 60 (40-63 μm). The silica gel layer was additionally washed by 100 ml of dichloromethane. The combined organic elute was evaporated to dryness to give 15.7 g (99%) of a white crystalline product which was further used without an additional purification.
Anal. calc. for $C_{29}H_{40}O$: C, 86.08; H, 9.96. Found: C, 86.26; H, 10.21.
$^1H$ NMR ($CDCl_3$): δ 7.36 (t, J = 1.8 Hz, 1 H, 4H in $C_6H_3{}^tBu_2$), 7.33 (d, J = 1.8 Hz, 2H, 2,6-H in $C_6H_3{}^tBu_2$), 7.21 (s, 1 H, 4-H in indenyl), 6.44 (m, 1 H, 3-H in indenyl), 3.17 (s, 3H, OMe), 3.14 (s, 2H, 1-H in indenyl), 2.06 (s, 3H, 2-Me in indenyl), 1.44 (s, 9H, 5-$^t$Bu in indenyl), 1.35 (s, 18H, $^t$Bu in $C_6H_3{}^tBu_2$). $^{13}C\{^1H\}$ NMR ($CDCl_3$): δ 150.4, 145.2 (two resonances), 141.7, 140.9, 140.6, 137.3, 132.5, 126.9, 124.0, 120.1, 116.9, 60.2, 43.0, 35.2, 34.9, 31.5, 31.0, 16.7.

**2-Methyl-3,5,6,7-tetrahydro-s-indacen-1(2*H*)-one**

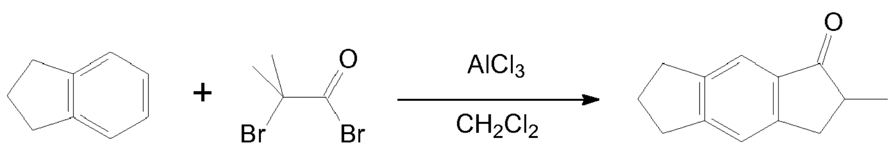


242 g (1.05 mol) of 2-bromo-2-methylpropionyl bromide was added dropwise over 15 min to a suspension of 333 g (2.5mol) of $AlCl_3$ in 900 ml of dichloromethane cooled to -30°C. The resulting mixture was stirred for 15 min, and then 118 g (1.0 mol) of indane was added at the same temperature. The cooling bath was then removed, and the solution was stirred overnight at room temperature. The reaction mixture was poured into 2 kg of crushed ice, the organic phase was separated, and the aqueous phase was extracted by 3 x 500 ml of dichloromethane. The combined organic extract was washed by aqueous $K_2CO_3$, dried over $K_2CO_3$, passed through a short pad of silica gel 60 (40-63 μm). The elute was evaporated to dryness to give a yellow oil. This oil was distilled in vacuum to give 145 g (78%) of a slightly yellowish oil, b.p. 120-140°C/5 mm Hg. The so obtained 2-methyl-3,5,6,7-tetrahydro-*s*-indacen-1(2*H*)-one contaminated with ca. 15% of the angular isomer, i.e. 2-methyl-1,6,7,8-tetrahydro-*as*-indacen-3(2*H*)-one, was used without additional purification.
Anal. calc. for $C_{13}H_{14}O$: C, 83.83; H, 7.58. Found: C, 83.74; H, 7.39.
$^1H$ NMR ($CDCl_3$): δ 7.54 (s, 1 H, 8-H), 7.24 (s, 1 H, 4-H), 3.30 (dd, *J* = 16.6 Hz, *J* = 7.3 Hz, 1 H, 3-C*H*H'), 2.84-3.00 (m, 4H, 5-$CH_2$ and 7-$CH_2$), 2.63-2.74 (m, 1 H, 2-H), 2.63 (dd, *J* = 16.6 Hz, *J* = 3.6 Hz, 1 H, 3-CH*H'*), 2.10 (tt, 2H, 6-$CH_2$), 1.28 (d, *J* = 7.4 Hz, 3H, 2-Me). $^{13}C\{^1H\}$ NMR ($CDCl_3$): δ 208.84, 152.87, 152.50, 144.05, 135.06, 121.94, 119.10, 42.36, 34.65, 33.01, 31.95, 25.70, 16.40.

**4,8-Dibromo-2-methyl-3,5,6,7-tetrahydro-s-indacen-1(2*H*)-one**

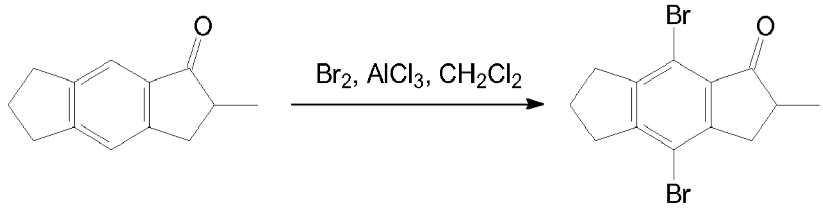


A solution of 141.7 g (760.8 mmol) of 2-methyl-3,5,6,7-tetrahydro-s-indacen-1(2*H*)-one (as prepared above, containing ca. 15% of the angular isomer) in 430 ml of dichloromethane was added dropwise for 0.5 h to a suspension of 260 g (1.95 mol, 2.56 eq.) of $AlCl_3$ in 700 ml of dichloromethane at -10°C. The reaction mixture was stirred for 10 min at this temperature, and then 1.3 g of iron powder was added. Further on, 250 g (1.56 mol, 2.06 eq.) of bromine was added dropwise for 1 h. The resulting mixture was stirred overnight at room temperature and then poured onto 2000 $cm^3$ of crushed ice. The organic layer was separated; the aqueous layer was extracted with 3 x 300 ml of dichloromethane. The combined organic extract was washed with aqueous $K_2CO_3$, dried over $K_2CO_3$, passed through a short pad of silica gel 60 (40-63 μm), and then evaporated to dryness. The crude product (ca. 264 g) was recrystallised from 3000 ml of hot n-hexane to yield the title product of ca. 95% purity. This material was further recrystallized from 2400 ml of hot n-hexane. This procedure gave 117 g of 4,8-dibromo-2-methyl-3,5,6,7-tetrahydro-s-indacen-1(2H)-one. The mother liquors were evaporated to dryness, and one more portion of the title product was isolated from the residue by flash chromatography on silica gel 60 (40-63 μm). This procedure gave 109 g of 4,8-dibromo-2-methyl-3,5,6,7-tetrahydro-s-indacen-1(2*H*)-one and 29.2 g of the angular isomeric product, i.e. 4,5-dibromo-2-methyl-1,6,7,8-tetrahydro-as-indacen-3(2*H*)-one. Thus, the total yield of the title product was 226 g (87%).
4,8-Dibromo-2-methyl-3,5,6,7-tetrahydro-*s*-indacen-1(2*H*)-one.
Anal. calc. for $C_{13}H_{12}Br_2O$: C, 45.38; H, 3.52. Found: C, 45.64; H, 3.60.
$^1H$ NMR ($CDCl_3$): δ 3.23 (dd, *J* = 17.6 Hz, *J* = 8.0 Hz, 1H, 3-C*H*H'), 3.04-3.12 (m, 4H, 5-$CH_2$ and 7-$CH_2$), 2.76 (m, 1H, 2-H), 2.54 (dd, *J* = 17.6 Hz, *J* = 3.7 Hz, 1H, 3-CH*H*'), 2.18 (quin, 2H, 6-$CH_2$), 1.32 (d, *J* = 7.2 Hz, 3H, 2-Me). $^{13}C\{^1H\}$ NMR ($CDCl_3$): δ 205.53, 154.61, 152.68, 147.07, 133.89, 117.86, 115.50, 43.17, 35.72, 34.88, 34.69, 23.30, 16.43.
4,5-Dibromo-2-methyl-1,6,7,8-tetrahydro-as-indacen-3(2*H*)-one.
Found: C, 45.50; H, 3.77.
$^1H$ NMR ($CDCl_3$): δ 3.14 (dd, *J* = 17.4 Hz, *J* = 8.02 Hz, 1H, 3-C*H*H'), 3.06 (t, *J* = 7.63 Hz, 2H, 6-$CH_2$), 2.97 (br. t, *J* = 7.63 Hz, 2H, 8-$CH_2$), 2.74 (m, 1H, 2-H), 2.48 (dd, *J* = 17.4 Hz, *J* = 4.0 Hz, 1 H, 3-CH*H*'), 2.20 (quin, *J* = 7.63 Hz, 2H, 7-$CH_2$), 1.31 (d, *J* = 7.43 Hz, 3H, 2-Me). $^{13}C\{^1H\}$ NMR ($CDCl_3$): δ 205.13, 152.93, 150.21, 141.48, 133.91, 123.51, 119.50, 43.03, 36.86, 32.26, 31.20, 23.95, 16.48.

**4,8-Dibromo-1-methoxy-2-methyl-1,2,3,5,6,7-hexahydro-*s*-indacene**

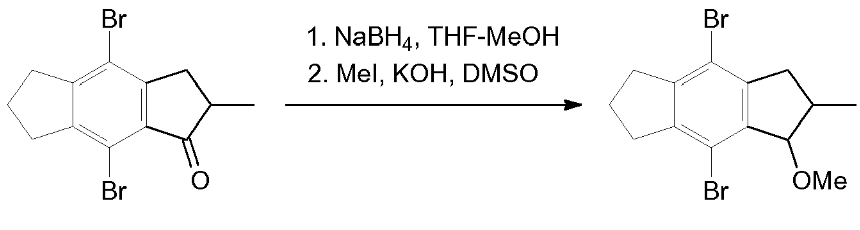


250 ml of methanol was added dropwise by vigorous stirring over 5 h to a mixture of 117 g (340 mmol) of 4,8-dibromo-2-methyl-3,5,6,7-tetrahydro-*s*-indacen-1(2*H*)-one and 19.3 g (0.51 mol) of $NaBH_4$ in 600 ml of THF at 0-5°C. This mixture was stirred overnight at room temperature and then evaporated to dryness. The residue was acidified by 2 M HCl to pH 5-6, and the formed 4,8-dibromo-2-methyl-1,2,3,5,6,7-hexahydro-*s*-indacen-1-ol was extracted with 1200 ml and then 2 x 200 ml of dichloromethane. The combined organic extract was dried over $Na_2SO_4$ and evaporated to dryness. The obtained white solid was dissolved in 800 ml of DMSO, 90 g (1.6 mol) of KOH and 110 g (0.775 mol) of methyl iodide was added. This mixture was stirred for 5 h at ambient temperature. The obtained solution was decanted from an excess of KOH, the latter was additionally washed by 3 x 350 ml of dichloromethane. The combined organic

extract was washed with 3000 ml of water. The organic layer was separated, and the aqueous layer was extracted with 3 x 300 ml of dichloromethane. The combined organic extract was washed with 7 x 1500 ml of water, dried over $Na_2SO_4$, and then evaporated to dryness. This procedure gave 121 g (99%) of 4,8-dibromo-1-methoxy-2-methyl-1,2,3,5,6,7-hexahydro-s-indacene as a colorless thick oil slowly crystallized at room temperature. The final material is a mixture of two stereoisomers.
Anal. calc. for $C_{14}H_{16}Br_2O$: C, 46.70; H, 4.48. Found: C, 47.02; H, 4.69.
*Syn*-isomer: $^1$H NMR ($CDCl_3$): δ 4.60 (d, *J* = 5.5 Hz, 1H, 1-H), 3.51 (s, 3H, OMe), 2.87-3.08 (m, 5H, 3-C*H*H', 5- and 7-$CH_2$), 2.74 (dd, *J* = 15.9 Hz, *J* = 9.7 Hz, 1H, 3-CH*H*'), 2.47 (m, 1 H, 2-H), 2.09 (quin, *J* = 7.4 Hz, 2H, 6-$CH_2$), 1.24 (d, *J* = 6.85 Hz, 3H, 2-Me). $^{13}$C{$^1$H} NMR ($CDCl_3$): δ 146.01, 144.83, 144.22, 143.06, 116.75, 116.22, 86.86, 59.05, 40.65, 39.29, 35.44, 35.38, 23.45, 13.56. *Anti*-isomer. $^1$H NMR ($CDCl_3$): δ 4.44 (s, 1H, 1-H), 3.43 (s, 3H, OMe), 3.31 (dd, *J* = 16.6 Hz, *J* = 7.2 Hz, 1H, 3-C*H*H'), 2.95-3.05 (m, 4H, 5- and 7-$CH_2$), 2.57 (m, 1H, 2-H), 2.46 (d, *J* = 16.6 Hz, 1H, 3-CH*H*'), 2.10 (quin, *J* = 7.6 Hz, 2H, 6-$CH_2$), 1.05 (d, *J* = 7.2 Hz, 3H, 2-Me). $^{13}$C{$^1$H} NMR ($CDCl_3$): δ 146.49, 144.67, 144.01, 140.71, 117.41, 116.70, 92.32, 56.83, 40.62, 36.89, 35.40, 35.23, 23.53, 19.81.

**4-Bromo-1-methoxy-2-methyl-1,2,3,5,6,7-hexahydro-*s*-indacene**

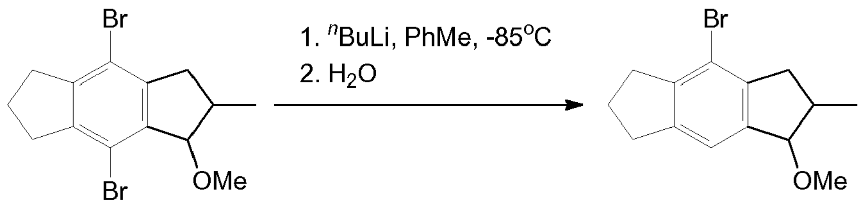


136 ml (340 mmol) of 2.5 M $^n$BuLi in hexanes was added dropwise over a period of 30 min to a solution of 120.3 g (334 mmol) of 4,8-dibromo-1-methoxy-2-methyl-1,2,3,5,6,7-hexahydro-s-indacene in 650 ml of toluene cooled to -85°C. The resulting mixture was allowed to warm over 1 h to -30°C and stirred at this temperature for 30 min. The reaction was quenched by 200 ml of water, yellowish organic layer was separated, and the aqueous layer was additionally extracted with 2 x 100 ml of dichloromethane. The combined organic extract was dried over $K_2CO_3$ and then passed through a short layer of silica gel 60 (40-63 μm). The silica gel layer was additionally washed with 50 ml of dichloromethane. The combined organic elute was evaporated to dryness, and the crude product was distilled under reduced pressure to give 87.2 g (92.9%) of 4-bromo-1-methoxy-2-methyl-1,2,3,5,6,7-hexahydro-*s*-indacene (bp 147-150°C/4 mm Hg) as a colorless liquid consisting of a mixture of two stereoisomers in a ca. 55:45 ratio.
Anal. calc. for $C_{14}H_{17}BrO$: C, 59.80; H, 6.09.Found: C, 59.99; H, 6.20.
$^1$H NMR ($CDCl_3$): δ 7.13 (s, 1H, 7-H), 7.12 (s, 1H, 7-H), 4.51 (d, *J* = 5.6 Hz, 1H, 1-H), 4.39 (d, *J* = 3.8 Hz, 1H, 1-H), 3.42 (s, 3H, OMe), 3.38 (s, 3H, OMe), 3.17 (dd, *J* = 16.4 Hz, *J* = 7.6 Hz, 1H, 3-C*H*H'), 2.97 (t, *J* = 7.4 Hz, 4H, 5- and 7-$CH_2$), 2.83 (m, 5H, 3-C*H*H', 5- and 7-$CH_2$), 2.55-2.69 (m, 2H, two 2-H), 2.51 (m, 1H, 3-CH*H*'), 2.38 (dd, *J* = 16.4 Hz, *J* = 4.8 Hz, 1 H, 3-CH*H*'), 2.08 (quin, *J* = 7.6 Hz, 4H, two 6-$CH_2$), 1.15 (d, *J* = 7.1 Hz, 3H, 2-Me), 1.09 (d, *J* = 6.8 Hz, 3H, 2-Me). $^{13}$C{$^1$H} NMR ($CDCl_3$): δ 144.63, 144.43, 144.30, 144.00, 142.69, 142.08, 141.50, 141.17, 119.93, 119.77, 117.68, 91.90, 86.54, 56.74, 56.33, 39.32, 39.07, 38.41, 34.06, 33.74, 24.70, 19.42, 13.58.

**4-(3,5-Di-*tert*-butylphenyl)-1-methoxy-2-methyl-1,2,3,5,6,7-hexahydro-*s*-indacene**

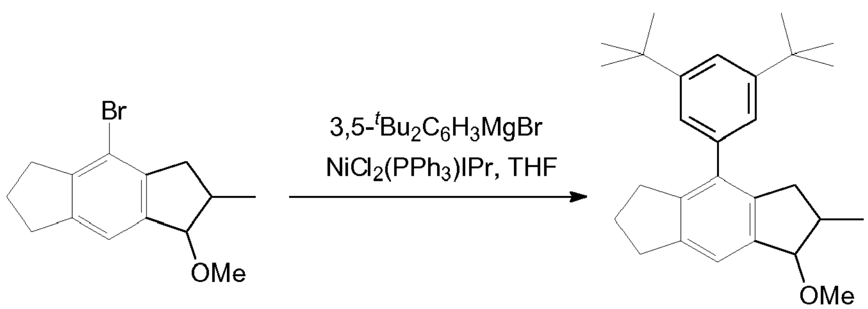

600 ml (270 mmol) of 0.45 M solution of 3,5-di-*tert*-butylphenylmagnesium bromide in THF was added in one portion to a mixture of 3.1 g (3.97 mmol) of $NiCl_2(PPh_3)$IPr and 56.4 g (201 mmol) of 4-bromo-1-methoxy-2-methyl-1,2,3,5,6,7-hexahydro-s-indacene. The resulting solution was refluxed for 2 hours. After cooling to room temperature, 150 ml of water was added to the reaction mixture and the main part of THF was distilled off in a rotary evaporator. 500 ml of dichloromethane and 1000 ml of 1 M HCl was added to the residue. Organic layer was separated and the aqueous layer was additionally extracted with 150 ml of dichloromethane. The combined organic extract was evaporated to dryness to give a red oil. The product was isolated by flash-chromatography on silica gel 60 (40-63 μm; eluent: hexanes-dichloromethane = 2:1, vol., then 1:1, vol.). This procedure gave 73.7 g (94%) of 4-(3,5-di-*tert*-butylphenyl)-1-methoxy-2-methyl-1,2,3,5,6,7-hexahydro-*s*-indacene as colorless thick oil as a mixture of two stereoisomers.
Anal. calc. for $C_{14}H_{17}BrO$: C, 59.80; H, 6.09.Found: C, 60.10; H, 6.23.
*Syn*-isomer: $^1$H NMR ($CDCl_3$): δ 7.34 (t, *J* = 1.6 Hz, 1H, 4-H in 3,5-$^t$Bu$_2$C$_6$H$_3$), 7.23 (s, 1H, 7-H in indenyl), 7.16 (d, *J* = 1.6 Hz, 2H, 2,6-H in 3,5-$^t$Bu$_2$C$_6$H$_3$), 4.49 (d, *J* = 5.5 Hz, 1H, 1-H in indenyl), 3.45 (s, 3H, OMe), 2.96 (t, *J* = 7.1 Hz, 2H), 2.6-2.92 (m, 4H), 2.54 (sept, *J* = 6.5 Hz, 1 H), 1.94-2.11 (m, 2H, 6-$CH_2$), 1.34 (s, 18H, 3,5-$^t$Bu$_2$C$_6$H$_3$), 1.09 (d, *J* = 6.85 Hz, 3H, 2-Me).
$^{13}$C{$^1$H} NMR ($CDCl_3$): δ 149.97, 142.82, 142.58, 141.62, 140.12, 138.66, 136.28, 123.46, 120.18, 120.02, 86.31, 56.76, 39.56, 37.78, 34.88, 33.12, 32.63, 31.53, 26.00, 13.69. *Anti*-isomer: $^1$H NMR ($CDCl_3$): δ 7.34 (t, *J* = 1.76 Hz, 1H, 4-H in 3,5-$^t$Bu$_2$C$_6$H$_3$), 7.24 (s, 1H, 7-H in indenyl), 7.16 (d, *J* = 1.76 Hz, 2H, 2,6-H in 3,5-$^t$Bu$_2$C$_6$H$_3$), 4.39 (d, *J* = 3.91 Hz, 1H, 1-H in indenyl), 3.49 (s, 3H, OMe), 3.15 (dd, *J* = 16 Hz, *J* = 7.5 Hz, 1H, C*H*H'), 2. 95 (t, *J* = 7.24 Hz, 2H, 5-$CH_2$), 2.72-2.91 (m, 2H, 7-$CH_2$), 2.41-2.53 (m, 1 H, 2-H), 2.3 (dd, *J* = 16 Hz, *J* = 4.8 Hz, 1H, CH*H'*), 1.95-2.11 (m, 2H, 6-$CH_2$), 1.34 (s, 18H, 3,5-$^t$*Bu*$_2$C$_6$H$_3$), 1.11 (d, *J* = 7.0 Hz, 3H, 2-Me). $^{13}$C{$^1$H} NMR ($CDCl_3$): δ 149.99, 143.29, 142.88, 140.91, 139.33, 138.62, 136.31, 123.39, 120.18, 120.01, 91.56, 56.45, 40.06, 37.89, 34.87, 33.09, 32.58, 31.52, 26.02, 19.31

**4-(3,5-Di-*tert*-butylphenyl)-6-methyl-1,2,3,5-tetrahydro-*s*-indacene**

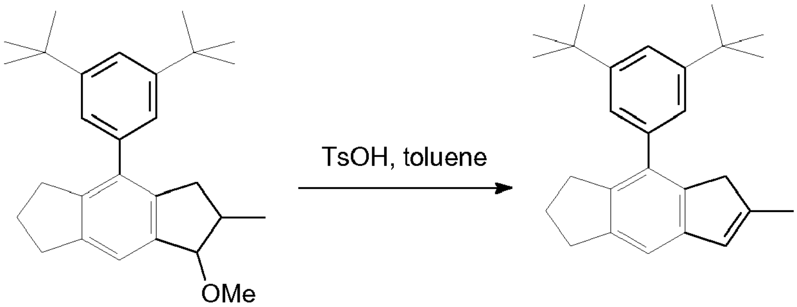


1.5 g of TsOH was added to a solution of 73.7 g (189 mmol) of 4-(3,5-di-*tert*-butylphenyl)-1-methoxy-2-methyl-1,2,3,5,6,7-hexahydro-s-indacene (as prepared above) in 700 ml of toluene, and the resulting solution was refluxed using Dean-Stark head for 15 min. After cooling to room temperature, the reaction mixture was washed with 200 ml of 10% aqueous $NaHCO_3$. The organic layer was separated, and the aqueous layer was additionally extracted with 2 x 150 ml of dichloromethane. The combined organic extract was evaporated to dryness to give a yellowish crystalline mass which was recrystallized from 250 ml of hot n-hexane to give 48.2 g of white crystalline product. The mother liquor was evaporated to dryness; the residue was recrystallized from 100 ml of n-hexane to give a second crop (13.3 g) of the product. Thus, the total yield of 4-(3,5-di-*tert*-butylphenyl)-6-methyl-1,2,3,5-tetrahydro-s-indacene isolated in this reaction was 61.5 g (91%).
Anal. calc. for $C_{27}H_{34}$: C, 90.44; H, 9.56. Found: C, 90.67; H, 9.74.
$^1$H NMR ($CDCl_3$): δ 7.45 (t, *J* = 1.76 Hz, 1 H, 4-H in 3,5-$^t$Bu$_2$C$_6$H$_3$), 7.33 (d, *J* = 1.76 Hz, 2H, 2,6-H in 3,5-$^t$Bu$_2$C$_6$H$_3$), 7.20 (s, 1 H, 8-H in indenyl), 6.56 (s, 1H, 7-H in indenyl), 3.28 (s, 2H, 5-$CH_2$), 3.06 (t, *J* = 7.2 Hz, 2H, 3-$CH_2$), 2.90 (t, *J* = 7.2 Hz, 2H, 1-$CH_2$), 2.17 (s, 3H, 6-$CH_2$), 2.13 (quin, *J* = 7.2 Hz, 2H, 2-$CH_2$), 1.44 (s, 18H, 3,5-$^t$Bu$_2$C$_6$H$_3$). $^{13}$C{$^1$H} NMR ($CDCl_3$): δ 150.17, 145.58, 144.91, 143.02, 139.85, 139.15, 138.01, 135.26, 127.07, 123.19, 120.24, 114.82, 42.23, 34.92, 33.29, 32.27, 31.56, 25.96, 16.80.

**Chloro[4-(3,5-di-*tert*-butylphenyl)-2-methyl-1,5,6,7-tetrahydro-s-indacen-1-yl]dimethylsilane**

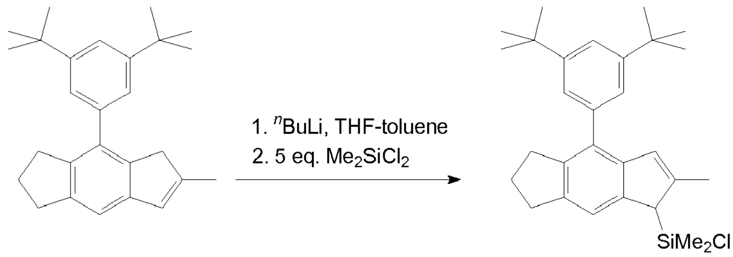


10.0 ml (25.0 mmol) of 2.5 M $^{n}$BuLi in hexanes was added at room temperature to a solution of 8.96 g (25.0 mmol) of 4-(3,5-di-*tert*-butylphenyl)-6-methyl-1,2,3,5-tetrahydro-*s*-indacene in a mixture of 200 ml of toluene and 7.5 ml of THF. This mixture was stirred for 2 h at 60°C. The resulting yellowish orange solution with a lot of yellow precipitate was cooled to -60°C, and 16.1 g (125 mmol, 5 eq.) of dichlorodimethylsilane was added in one portion. The resulting solution was stirred overnight at room temperature and then filtered through a glass frit (G3). The precipitate was additionally washed by 2 x 30 ml of toluene. The combined filtrate was evaporated to dryness to give chloro[4-(3,5-di-*tert*-butylphenyl)-2-methyl-1,5,6,7-tetrahydro-*s*-indacen-1-yl]dimethylsilane as a yellowish glass which was used without additional purification.

$^{1}$H NMR ($CDCl_3$): δ 7.39 (t, *J* = 1.76 Hz, 1 H, 4-H in 3,5-$^{t}Bu_2C_6H_3$), 7.32 (s, 1 H, 8-H in indenyl), 7.25 (d, *J* = 1.76 Hz, 2H, 2,6-H in 3,5-$^{t}Bu_2C_6H_3$), 6.60 (s, 1H, 3-H in indenyl), 3.59 (s, 1 H, 1-H in indenyl), 2.94-3.08 (m, 2H, 7-$CH_2$), 2.83-2.99 (m, 2H, 5-$CH_2$), 2.33 (s, 3H, 2-Me in indenyl), 2.07 (quin, *J* = 7.24 Hz, 2H, 6-$CH_2$), 1.39 (s, 18H, 3,5-$^{t}Bu_2C_6H_3$), 0.47 (s, 3H, Si*Me*Me'), 0.21 (s, 3H, SiMe*Me*'). $^{13}$C{$^{1}$H} NMR ($CDCl_3$): δ 150.02, 144.41, 142.13, 141.54, 139.92 (two resonances), 138.78, 131.41, 127.01, 123.94, 120.14, 118.64, 49.78, 34.89, 33.32, 32.51, 31.57, 26.04, 17.72, 1.26, -0.53.

**[6-*tert*-Butyl-4-(3,5-di-*tert*-butylphenyl)-5-methoxy-2-methyl-1*H*-inden-1-yl][4-(3,5-di-*tert*-butylphenyl)-2-methyl-1,5,6,7-tetrahydro-s-indacen-1-yl]dimethylsilane**

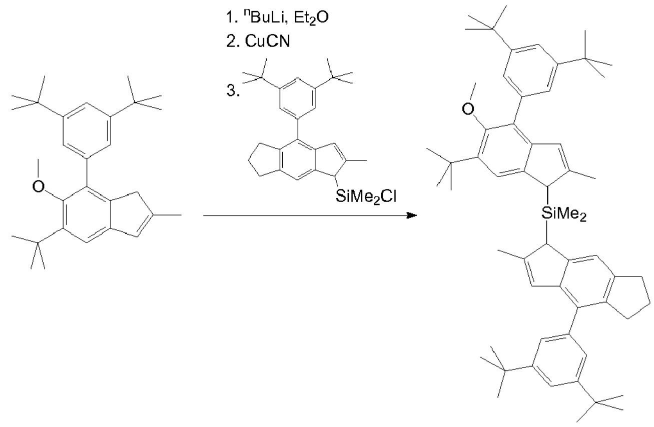


10.0 ml (25 mmol) of 2.5 M $^{n}$BuLi in hexanes was added in one portion to a solution of 10.1 g (25 mmol) of 5-*tert*-butyl-7-(3,5-di-*tert*-butylphenyl)-6-methoxy-2-methyl-1*H*-indene in 200 ml of ether at -50°C. This mixture was stirred overnight at room temperature, then the resulting yellow suspension was cooled to -50°C, and 250 mg of CuCN was added. The obtained mixture was stirred for 30 min at -25°C, then a solution of chloro[4-(3,5-di-*tert*-butylphenyl)-2-methyl-1,5,6,7-tetrahydro-s-indacen-1-yl]dimethylsilane (as prepared above, -25 mmol) in 200 ml of ether was added in one portion. The formed mixture was stirred overnight at ambient temperature, then filtered through a pad of silica gel 60 (40-63 μm) which was additionally washed with 2 x 50 ml of dichloromethane. The combined filtrate was evaporated to dryness, and the residue was dried in vacuum at elevated temperature. This procedure gave 19.8 g (97%) of a yellowish glass of [6-*tert*-butyl-4-(3,5-di-tert-butylphenyl)-5-methoxy-2-methyl-1*H*-inden-1-yl][4-(3,5-di-*tert*-butylphenyl)-2-methyl-1,5,6,7-tetrahydro-s-indacen-1-yl]dimethylsilane (>90% purity on the evidence of NMR spectroscopy, a ca. 1:1 mixture of the stereoisomers) which was further used without an additional purification.

$^{1}$H NMR ($CDCl_3$): δ 7.51 (s), 7.33-7.42 (m), 7.22-7.31 (m), 6.60 (s), 6.53 (s), 3.74 (s), 3.70 (s), 3.68 (s), 3.21 (s), 3.19 (s), 2.83-3.03 (m), 2.22 (s), 2.19 (s), 1.99-2.11 (m), 1.45 (s), 1.43 (s), 1.36 (s), -0.16 (s), -0.17 (s), -0.21 (s).

**Dimethylsilanediyl[η[5]-6-*tert*-butyl-4-(3,5-di-*tert*-butylphenyl)-5-methoxy-2-methylinden-1-yl][η[5]-4-(3,5-di-*tert*-butylphenyl)-2-methyl-5,6,7-trihydro-*s*-indacen-1-yl]zirconium dichloride**

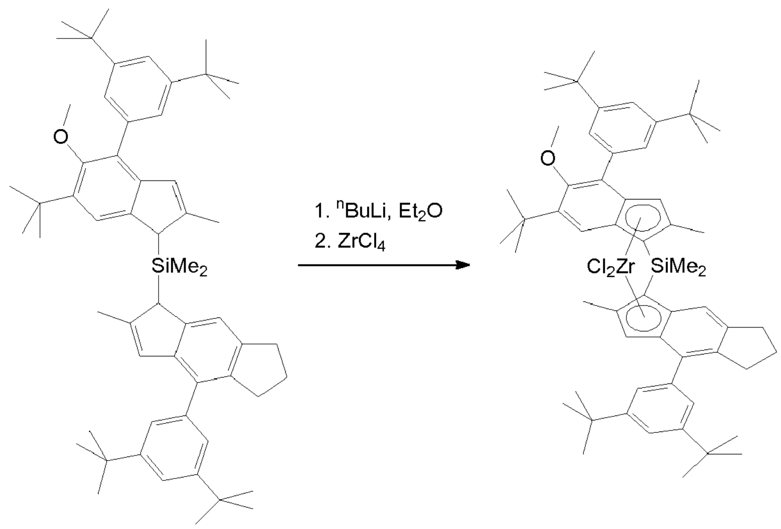


19.3 ml (48.3 mmol) of 2.5 M $^n$BuLi in hexanes was added in one portion to a solution of 19.8 g (24.1 mmol) of [6-*tert*-butyl-4-(3,5-di-*tert*-butylphenyl)-5-methoxy-2-methyl-1*H*-inden-1-yl][4-(3,5-di-*tert*-butylphenyl)-2-methyl-1,5,6,7-tetrahydro-s-indacen-1-yl]dimethylsilane (as prepared above) in 300 ml of ether at -50°C. This mixture was stirred overnight at room temperature. The resulting light-orange solution was cooled to -50°C, and then 5.63 g (24.2 mmol) of $ZrCl_4$ was added. This mixture was stirred for 24 h at room temperature. The resulting orange suspension was evaporated to dryness. The residue was dissolved in 250 ml of warm toluene, and the resulting hot suspension was filtered through a glass frit (G4). On the evidence of NMR spectroscopy the obtained filtrate included a ca. 1 to 1 mixture of *anti*- and *syn*-zirconocenes. This filtrate was concentrated to ca. 90 ml. The pale orange crystalline solid precipitated from this solution overnight at room temperature was filtered off, washed with 2 x 20 ml of toluene, then 2 x 20 ml of n-hexane, and dried in vacuum. This procedure gave 4.23 g of a ca. 83 to 17 mixture of *anti*- and *syn*-zirconocenes as toluene monosolvates. The mother liquor was further evaporated to ca. 60 ml. The reddish solid precipitated from this solution for 3 h at room temperature was filtered off and dried in vacuum. This procedure gave 2.48 g of *syn*-zirconocene as toluene monosolvate. The mother liquor was evaporated to ca. 45 ml. The reddish solid precipitated from this solution for 1 h was filtered off and dried in vacuum. This procedure gave 3.52 g of a ca. 93 to 7 mixture of *syn*- and *anti*-zirconocenes as toluene monosolvates. Again, the mother liquor was evaporated to ca. 35 ml. Pale orange solid precipitated from this solution overnight at room temperature was filtered off and dried in vacuum. This procedure gave 4.72 g of *anti*-zirconocene as toluene monosolvate. Thus, the total yield of *anti*- and *syn*-zirconocenes (as toluene monosolvates) isolated in this synthesis was 14.95 g (58%).

*Anti*-isomer.

Anal. calc. for $C_{58}H_{76}Cl_2OSiZr \times C_7H_8$: C, 72.85; H, 7.90. Found: C, 73.04; H, 8.08.
$^1$H NMR ($CD_2Cl_2$, -20°C): δ 7.70 (br.s, 1 H), 7.60 (s, 1 H), 7.50 (s, 1 H), 7.43 (s, 1 H), 7.35-7.39 (m, 2H), 7.33 (t, *J* = 1.84 Hz, 1H), 7.26 (s, 1H), 6.75 (s, 1H), 6.59 (s, 1H), 3.30 (s, 3H), 3.09-3.17 (m, 1H), 2.91-3.00 (m, 2H), 2.78-2.85 (m, 1H), 2.18 (s, 3H), 2.16 (s, 3H), 2.03-2.12 (m, 1H), 1.90-2.00 (m, 1H), 1.39 (s, 9H), 1.31-1.37 (m, 27H), 1.30 (s, 3H), 1.28 (s, 3H), 1.28 (s, 9H). $^{13}C\{^1H\}$ NMR ($CD_2Cl_2$, -20°C): δ 159.78, 150.82, 150.67, 150.06, 149.53, 144.49, 143.69, 142.90, 137.35, 135.70, 135.03, 133.54, 133.48, 132.88, 132.56, 127.36, 126.94, 124.67, 124.41, 124.03, 123.22, 122.90, 121.62, 121.02, 120.61, 120.55, 120.10, 117.81, 81.58, 81.01, 62.42, 35.68, 35.10, 34.98, 34.82, 33.12, 32.37, 31.48, 31.38, 30.29, 26.58, 18.38, 2.62, 2.54.[1] Resonances attributed to toluene were removed from this description of the NMR spectra.
[1] Resonances of some carbons in the aliphatic region coincided.

*Syn*-isomer.

Found: C, 73.15; H, 8.13.

$^1H$ NMR ($CD_2Cl_2$, -20°C): δ 7.82 (br.s, 1H), 7.71 (s, 1H), 7.51 (s, 1H), 7.41 (s, 1H), 7.35 (t, *J* = 1.84 Hz, 1H), 7.33 (t, *J* = 1.84 Hz, 1H), 7.29 (s, 1H), 7.24 (br.s, 1H), 6.74 (s, 1H), 6.53 (s, 1 H), 3.11 (s, 3H), 3.04-3.10 (m, 1H), 2.76-2.91 (m, 3H), 2.39 (s, 3H), 2.37 (s, 3H), 1.99-2.06 (m, 1 H), 1.63-1.75 (m, 1 H), 1.44 (s, 3H), 1.38 (br.s, 9H), 1.34 (s, 9H), 1.33 (s, 9H), 1.32 (s, 9H), 1.31 (br.s, 9H), 1.21 (s, 9H). $^{13}C\{^1H\}$ NMR ($CD_2Cl_2$, -20°C): δ 158.77, 150.64, 150.10, 149.61, 143.44, 142.74, 141.74, 136.87, 136.30, 135.68, 135.29, 135.17, 134.33, 131.59, 126.50, 124.38, 124.08, 124.03, 123.65, 123.36, 121.55, 121.04, 120.90, 120.84, 120.15, 118.34, 82.86, 82.72, 62.12, 35.44, 35.12, 34.97, 34.79, 33.17, 32.48, 31.45, 31.42, 30.13, 26.77, 18.63, 18.55, 2.87, 2.68. [2] Resonances attributed to toluene were removed from this description of NMR spectra.
[2] Resonances of some carbons coincided.

**b) Catalyst system**

**Example 1:**

The complex C-1 was used

*Step 1.*

Inside the glovebox, 80 μL of dry and degassed FluorN 474 were mixed with 2 mL of MAO in a septum bottle and left to react overnight. The following day, 68.80 mg of metallocene C1 (0,076 mmol, 1 equivalent) were dissolved with 4 mL of the MAO solution in another septum bottle and left to stir inside the glovebox.
After 60 minutes, 1 mL of the surfactant solution and the 4 mL of the MAO-metallocene solution were successively added into a 50 mL emulsification glass reactor containing 40 mL of PFC at -10 °C and equipped with an overhead stirrer (stirring speed = 600 rpm). *Total amount of MAO is 5 mL (300 equivalents).* A red emulsion formed immediately and stirred during 15 minutes at -10 °C / 600 rpm. Then the emulsion was transferred via a 2/4 teflon tube to 100mL of hot PFC at 90 °C, and stirred at 600 rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining catalyst was dried during 2 hours at 50 °C over an argon flow. 0.77 g of a red solid catalyst was obtained (Al-wt% = 31.0 and Zr-wt% = 0.34).

*Step 2.*

*Off-line prepolymerization procedure*

Off-line pre-polymerization experiments were done in a 125 mL pressure reactor equipped with gas-feeding lines and an overhead stirrer. Dry and degassed perfluoro-1.3-dimethylcyclohexane (15 $cm^3$) and 0.6865 g of the catalyst produced in the step 1, to be pre-polymerized, were loaded into the reactor inside a glove box and the reactor was sealed. The reactor was then taken out from the glove box and placed inside a water cooled bath kept at 25 °C. The overhead stirrer and the feeding lines were then connected. The experiment was started by opening the propylene feed into the reactor. The propylene feed was left open and the monomer consumption was compensated by keeping the total pressure in the reactor constant (5 bar). The experiment was continued for the polymerization time (15 min) sufficient to provide the desired degree of polymerization (DP 3.9). The reactor was then taken back inside the glove box before opening and the content was poured into a glass vessel. The perfluoro-1.3-dimethylcyclohexane was evaporated until a constant weight was obtained to yield 3.3426 g of the pre-polymerized catalyst.

**Example 2:**

Complex 1 was used:

Inside the glovebox, 103.21 of metallocene C1 was mixed with 5 ml MAO (30wt% in toluene) in a septum bottle and the solution was stirred for 60 minutes and then 105.15 mg of tritylBF20 was added. The mixture was left to react overnight at room temperature inside the glovebox.

**Example 3:**

Complex 2 was used

Inside the glovebox, 111,65 mg of metallocene C2 was mixed with 5 ml MAO (30wt% in toluene) in a septum

bottle and the solution was stirred for 60 minutes and then 105.15 mg of tritylBF20 was added. The mixture was left to react overnight at room temperature inside the glovebox.

**Table 1:** Catalyst Composition

| cat. | Metallocene | DofP[1] | Al/Zr[2] | B/Zr[3] |
|---|---|---|---|---|
| | | [g/g] | [mol/mol] | [mol/mol] |
| Ex 1 | C1 | 3.9 | 300 | 0.0 |
| Ex 2 | C1 | n.a | 200 | 1.0 |
| Ex 3 | C2 | n.a | 200 | 1.0 |

[1] Degree of off-line pre-polymerization
[2] Al/Zr molar ratio in catalyst
[3] B/Zr molar ratio in catalyst
n.a not applicable

**Polymerization**

In order to prove the suitability of the catalyst systems according to the present invention two kind of polymerizations were performed.
In Example 4 the polymerization reaction was carried out in a 480 mL pressure reactor at 110°C.
In Examples 5 and 6 a polymerization reaction was carried out in Parallel Polymerization Reactors (PPR) (provided by Symyx) (10 mL Reactor Volume) at 190°C

**Example 4**

**The catalyst of Example 1 was used (C1 activated with MAO only)**

ethylene/1-octene solution polymerizations was performed according to the following procedure in heptane at 110°C without $H_2$.
First 1-octene was fed into the reactor by means of a Waters HPLC pump in the desired amounts, then 200 mL heptane by means of 10 mL syringe. The stirring speed was set to 150 rpm. 50 μmol of triethylaluminium (TEA) (as a scavenger) as a 0.5 moL/L solution in heptane was fed into the reactor. The reactor temperature was set to 110°C. The reactor was fed with ethylene up to the required pressure (P = 20 bar) and the desired catalyst amount was injected at the polymerization temperature. The pressure was kept constant, feeding ethylene and after 20min polymerization time the catalyst was killed by air addition and venting the reactors. The samples were stabilized with 2500 ppm Irganox B225 (dissolved in acetone).

**Table 2:** results for ethylene/1-octene solution co-polymerization

| | |
|---|---|
| Cat amount [mg] | 20 |
| Used 1-octene [g] | 12 |
| Yield [g] | 2.0 |
| Productivity in 20 min [kg/g MC] | 10.2 |
| Ethylene/1-octene in liq. Phase [wt/wt] | 0.76 |
| 1-Octene incorporation [wt% NMR] | 27.7 |
| Tm [°C] | 84 |
| Tc [°C] | 65 |

**Polymerization procedure for Example 5 and 6:**

The vessels were loaded inside a glovebox utilizing a 3-axis liquid handling robot. A preweighed glass vial with stirring paddles was sealed and purged with nitrogen. A volume of about 4.1 mL of corresponding solvent (decane) was

filled in each PPR reactor. Then, 15 µmol of triethyl aluminium (TEA) as scavenger was added, along with 0,45 g of octene as comonomer at room temperature. The ethylene pressure was set to 10 bar to check any leaks. Then, the temperature and pressure had been increased to the set value (T = 190°C and P = 24 bar) and once the steady state was reached, the corresponding volume of pre-activated catalyst solution in toluene and 0.9 mL of dry-decane had been injected in the reactor to start the polymerization under mechanical stirring. The run was quenched with $CO_2$ after the set amount of ethylene uptake had been reached (20 min as a maximum run time). The glass vials had been dried by vacuum centrifuge and weighed.

**Table 4:** PPR experiments conditions for ethylene/1-octene solution co-polymerization

| Example | 5 | 6 |
|---|---|---|
| Complex | C1 | C2 |
| Catalyst system [µl] | 38.7 | 35.7 |
| Used 1-octene [g] | 0.45 | 0.45 |
| Decane [g] | 3.6 | 3.6 |
| Ethylene [g] | 0.45 | 0.45 |
| TEAL Scavenger [µmol] | 15.0 | 15.0 |

**Table 5:** PPR experiments results for ethylene/1-octene solution co-polymerization

| Ex. | MC | Cat amount [mg] | Quench time [min] | Productivity [kgPE/gMC] | Tm [°C] | Tc [°C] | 1-octene incorporation [wt% NMR] |
|---|---|---|---|---|---|---|---|
| 5 | C-1 | 0.07 | 7.8 | 5.5 | 96 | 81 | 22.0 |
| 6 | C-2 | 0.07 | 16.5 | 6.4 | 94 | 93 | 18.3 |

## Claims

**1.** Catalyst system for producing ethylene copolymers in a high temperature solution process, the catalyst system comprising

(i) a metallocene complex of formula (I)

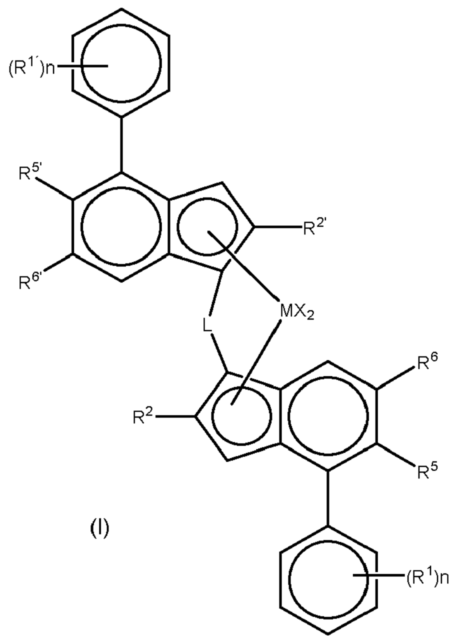

(I)

wherein

M is Hf or Zr
X is a sigma ligand
L is a bridge of the formula $-SiR^7_2-$, wherein each $R^7$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl
$R^1$ and $R^{1'}$ are the same or can be different and can be a linear or branched $C_1$-$C_6$-alkyl group
n is 0, 1 or 2
$R^2$ and $R^{2'}$ are the same or can be different and are a $CH_2$-$R^8$ group, with $R^8$ being H or linear or branched $C_1$-$C_6$-alkyl group
$R^5$ and $R^{5'}$ are the same or are different and can be a linear or branched $C_1$-$C_6$-alkyl group or a OR group, wherein R is a $C_1$-$C_6$-alkyl group
$R^6$ and $R^{6'}$ are the same or are different and can be a $C(R^9)_3$ group, with $R^9$ being the same or different and $R^9$ can be H or a linear or branched $C_1$-$C_6$-alkyl group
or $R^5$ and $R^6$ and/or $R^{5'}$ and $R^{6'}$ taken together form an unsubstituted 4-7 membered ring condensed to the benzene ring of the indenyl moiety,

with the proviso that if $R^5$ and $R^6$ as well as $R^{5'}$ and $R^{6'}$ taken together form an unsubstituted 5 membered ring condensed to the benzene ring of the indenyl moiety then $R^2$ and $R^{2'}$ are not a $CH_3$ group;
(ii) an aluminoxane cocatalyst and
(iii) optionally a boron containing cocatalyst

**2.** Catalyst system according to claim 1, wherein in the formula (I)
M is Zr,
X is which may be the same or different, and is a hydrogen atom, a halogen atom, a $R^{10}$, $OR^{10}$, $OSO_2CF_3$, $OCOR^{10}$, $SR^{10}$, $NR^{10}_2$ or $PR^{10}_2$ group, wherein $R^{10}$ is a linear or branched, cyclic or acyclic, $C_1$-$C_{20}$-alkyl, $C_2$-$C_{20}$-alkenyl, $C_2$-$C_{20}$-alkynyl, $C_6$-$C_{20}$-aryl, $C_7$-$C_{20}$-alkylaryl or $C_7$-$C_{20}$-arylalkyl radical; optionally containing heteroatoms belonging to groups 14-16 or is $SiR^{10}_3$, $SiHR^{10}_2$ or $SiH_2R^{10}$,
L is a bridge of the formula $-SiR^7_2-$, wherein both $R^7$ are the same $C_1$-$C_{10}$-hydrocarbyl or $C_6$-$C_{10}$-aryl group,
$R^1$ and $R^{1'}$ are the same and are a linear or branched $C_1$-$C_6$-alkyl group,
n is 1 or 2,
$R^2$ and $R^{2'}$ are the same and are a $CH_2$-$R^8$ group, with $R^8$ being H or linear or branched $C_1$-$C_4$-alkyl group
$R^5$ and $R^{5'}$ are the same or are different and can be a OR group, wherein R is a $C_1$-$C_4$-alkyl group
$R^6$ and $R^{6'}$ are the same or are different and can be a $C(R^9)_3$ group, with $R^9$ being the same or different and $R^9$ can be a linear or branched $C_1$-$C_4$-alkyl group
or $R^5$ and $R^6$ and/or $R^{5'}$ and $R^{6'}$ taken together form an unsubstituted 5-6 membered ring condensed to the benzene ring of the indenyl moiety,
with the proviso that if $R^5$ and $R^6$ as well as $R^{5'}$ and $R^{6'}$ taken together form an unsubstituted 5 membered ring condensed to the benzene ring of the indenyl moiety then $R^2$ and $R^{2'}$ are not a $CH_3$ group.

**3.** Catalyst system according to claim 1 or 2, wherein in the formula (I)
M is Zr,
X is independently a hydrogen atom, a halogen atom, $C_{1-6}$-alkoxy group or an $R^{10}$ group, wherein $R^{10}$ a $C_{1-6}$-alkyl, phenyl or benzyl group,
L is a bridge of the formula $-SiR^7_2-$, wherein both $R^7$ are the same $C_1$-$C_4$-hydrocarbyl or $C_6$-aryl group,
$R^1$ and $R^{1'}$ are the same and are a linear or branched $C_1$-$C_4$-alkyl group,
n is 1 or 2,
$R^2$ and $R^{2'}$ are the same and are a $CH_2$-$R^8$ group, with $R^8$ being H or $C_1$-$C_4$-alkyl group,
one of $R^5$ and $R^6$ or $R^{5'}$ and $R^{6'}$ form together an unsubstituted 5-6 membered ring condensed to the benzene ring of the indenyl moiety,
and the remaining residues of $R^5$ and $R^6$ or $R^{5'}$ and $R^6$, are for $R^5$ or $R^{5'}$ a OR group, wherein R is a $C_1$-$C_4$-alkyl group and for $R^6$ or $R^{6'}$ a $C(R^9)_3$ group, with $R^9$ being the same and $R^9$ can be a $C_1$-$C_2$-alkyl group.

**4.** Catalyst system according to claim 1 or 2, wherein in the formula (I)
M is Zr,
X is independently a hydrogen atom, a halogen atom, $C_{1-6}$-alkoxy group or an $R^{10}$ group, wherein $R^{10}$ a $C_{1-6}$-alkyl, phenyl or benzyl group,
L is a bridge of the formula $-SiR^7_2-$, wherein both $R^7$ are the same $C_1$-$C_4$-hydrocarbyl or $C_6$-aryl group,

$R^1$ and $R^{1'}$ are the same and are a linear or branched $C_1$-$C_4$-alkyl group,
n is 1 or 2,
$R^2$ and $R^{2'}$ are the same and are a $CH_2$-$R^8$ group, with $R^8$ being linear or branched $C_1$-$C_3$-alkyl group
$R^5$ and $R^6$ and $R^{5'}$ and $R^{6'}$ form together an unsubstituted 5-6 membered ring condensed to the benzene ring of the indenyl moiety

**5.** Catalyst system according to anyone of the preceding claims, wherein the metallocene complex of formula (I) is *racemic* dimethylsilanediylbis[2-*iso*-butyl-4-(4-*tert*-butylphenyl)-5,6,7-trihydro-s-indacen-1-yl] zirconium dichloride or dimethyl,
racemic dimethylsilanediyl[η5-6-*tert*-butyl-4-(3,5-di-*tert*-butylphenyl)-5-methoxy-2-methyl-inden-1-yl][η5-4-(3,5-di-*tert*-butylphenyl)-2-methyl-5,6,7-trihydro-s-indacen-1-yl] zirconium dichloride or dimethyl, either in their syn or anti configuration.

**6.** Metallocene complex of formula (I), wherein
M is Zr,
X is Cl or methyl group,
L is a bridge of the formula $-SiR^7_2-$, wherein both $R^7$ are the same $C_1$-$C_4$-hydrocarbyl or $C_6$-aryl group,
$R^1$ and $R^{1'}$ are the same and are a linear or branched $C_1$-$C_4$-alkyl group,
n is 1 or 2,
$R^2$ and $R^{2'}$ are the same and are a $CH_2$-$R^8$ group, with $R^8$ being H or a $C_1$-$C_3$-alkyl group, one of $R^5$ and $R^6$ or $R^{5'}$ and $R^{6'}$ form together an unsubstituted 5-6 membered ring condensed to the benzene ring of the indenyl moiety, and the remaining residues of $R^5$ and $R^6$ or $R^{5'}$ and $R^6$, are for $R^5$ or $R^{5'}$ a OR group, wherein R is a $C_1$-$C_4$-alkyl group and for $R^6$ or $R^{6'}$ a $C(R^9)_3$ group, with $R^9$ being the same and $R^9$ can be a $C_1$-$C_2$-alkyl group.

**7.** Catalyst system according to any of preceding claims, being obtainable by a process in which

(a) a liquid/liquid emulsion system is formed, said liquid/liquid emulsion system comprising a solution of the catalyst components (i) to (iii) dispersed in a solvent so as to form dispersed droplets; and
(b) solid particles are formed by solidifying said dispersed droplets.

**8.** A catalyst system according to claim 7 wherein the solid particles are prepolymerized in a step (c).

**9.** Catalyst system according to any of preceding claims, being a non-supported catalyst systems obtainable by contacting the metallocene of formula (I) as a solid or as a solution with the cocatalyst(s) previously dissolved in an aromatic solvent, or being obtainable by sequentially adding the catalyst components to the polymerization medium.

**10.** A catalyst system as claimed in any preceding claim wherein said aluminoxane cocatalyst is MAO.

**11.** A catalyst system as claimed in any preceding claim wherein said optional boron containing cocatalyst comprises an anion of formula:

$$(Z)_4B^- \qquad (III)$$

where Z is an optionally substituted phenyl derivative, said substituent being a halo-$C_{1-6}$-alkyl or halo group, preferably
triphenylcarbeniumtetrakis(pentafluorophenyl) borate,
N,N-dimethylcyclohexylammoniumtetrakis(pentafluorophenyl)borate,
N,N- dimethylbenzylammoniumtetrakis(pentafluorophenyl)borate, or
N,N-dimethylaniliniumtetrakis(pentafluorophenyl)borate.

**12.** Use in olefin polymerization of a catalyst as defined in claim 1 to 11, in a high temperature solution process at a temperature greater than 100°C for polymerizing ethylene and a C4-10 alpha-olefin comonomer to produce polyethylene with

a) a comonomer content (measured with NMR) up to 40 wt%, preferably between 5 to 40 wt%,
b) a density (measured according to ISO 1183-187) of the in the range of 0.850 g/cm$^3$ to 0.950 g/cm$^3$, preferably in the range of 0.850 g/cm$^3$ to 0.945 g/cm$^3$,
c) a Mw/Mn value (measured with GPC) of the polymers of the invention is less than 5, preferably in the range

of 2.0 to 4.5. and
d) a melting points (measured with DSC according to ISO 11357-3:1999) below 130°C, preferably below 120°C.

**13.** Use according to claim 12, wherein the comonomer is butene, hexene or octene.

**14.** Use according to claim 12, wherein the comonomer octene.

**15.** Process for the preparation of an ethylene copolymer comprising polymerizing ethylene and a $C_{4-10}$ alpha-olefin comonomer in a high temperature solution process at a temperature greater than 100°C in the presence of a catalyst comprising:

(i) a metallocene complex of formula (I) as defined in any of preceding claims 1 to 11
(ii) an aluminoxane cocatalyst and
(iii) optionally a boron containing cocatalyst.

**16.** Process according to claim 15, wherein the polymerization is performed

a) at a polymerization temperature of at least 110°C,
b) a pressure in the range of 10 to 100 bar and
c) in a liquid hydrocarbon solvent selected from the group of $C_{5-12}$-hydrocarbons, which may be unsubstituted or substituted by $C_{1-4}$ alkyl group

Europäisches Patentamt
European Patent Office
Office européen des brevets

# EUROPEAN SEARCH REPORT

Application Number
EP 14 16 5140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/065844 A2 (EXXONMOBIL CHEM PATENTS INC [US]; VOSKOBOYNIKOV ALEXANDER Z [RU]; RYAB) 22 June 2006 (2006-06-22)<br>* page 260; example 13i2;13i *<br>----- | 1,2 | INV.<br>C08F210/16<br>C08F4/6592<br>C07F15/00 |
| X<br><br><br>Y | WO 2012/001052 A2 (BOREALIS AG [AT]; CASTRO PASCAL [FI]; RESCONI LUIGI [IT]; HUHTANEN LAU) 5 January 2012 (2012-01-05)<br>* page 50 - page 56; example 1; tables 1-4 *<br>----- | 4,7-16<br><br><br>3-16 | |
| Y | ILYA E. NIFANT'EV ET AL: "5-Methoxy-Substituted Zirconium Bis-indenyl ansa -Complexes: Synthesis, Structure, and Catalytic Activity in the Polymerization and Copolymerization of Alkenes", ORGANOMETALLICS, vol. 31, no. 14, 23 July 2012 (2012-07-23), pages 4962-4970, XP55060553, ISSN: 0276-7333, DOI: 10.1021/om300160v<br>* page 4964; examples 13,14 *<br>* page 4965, right-hand column - page 4966, right-hand column; figure 2; tables 2-3 *<br>* see conclusions; page 4968, right-hand column *<br>----- | 3-16 | TECHNICAL FIELDS SEARCHED (IPC)<br>C08F<br>C07F |
| Y | WO 2013/029699 A1 (BOREALIS AG [AT]; TYNYS ANTTI [AT]; RUEMER FRANZ [AT]; MALM BO [FI]; M) 7 March 2013 (2013-03-07)<br>* page 11, line 30 - line 37 *<br>* claims 2,3 *<br>----- | 3-16 | |
| Y | EP 2 657 285 A1 (BOREALIS AG [AT]) 30 October 2013 (2013-10-30)<br>* paragraph [0145] - paragraph [0156] *<br>* example 2; table 1 *<br>----- | 3-16 | |
| | -/-- | | |

~~The present search report has been drawn up for all claims~~

3

| Place of search | Date of completion of the search | Examiner |
|---|---|---|
| The Hague | 22 January 2015 | Parry, Julian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 16 5140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2012/084961 A1 (BOREALIS AG [AT]; RESCONI LUIGI [IT]; CASTRO PASCAL [FI]; VOSKOBOYNIKO) 28 June 2012 (2012-06-28)<br>* page 57 - page 68, line 11; examples; tables *<br>----- | 3-16 | |
| Y | WO 2011/135005 A2 (BOREALIS AG [AT]; RESCONI LUIGI [FI]; CASTRO PASCAL [FI]; HUHTANEN LAU) 3 November 2011 (2011-11-03)<br>* tables 1-6 *<br>----- | 3-16 | |
| Y,D | WO 2011/135004 A2 (BOREALIS AG [AT]; RESCONI LUIGI [FI]; CASTRO PASCAL [FI]; HUHTANEN LAU) 3 November 2011 (2011-11-03)<br>* tables 1-3 *<br>----- | 3-16 | |
| Y | WO 2007/116034 A1 (BASELL POLYOLEFINE GMBH [DE]; RESCONI LUIGI [IT]; FOCANTE FRANCESCA [I) 18 October 2007 (2007-10-18)<br>* examples; tables 1-3 *<br>* page 1, line 17 - line 20 *<br>----- | 3-16 | |
| | | | TECHNICAL FIELDS SEARCHED (IPC) |

~~The present search report has been drawn up for all claims~~

3

| Place of search | Date of completion of the search | Examiner |
|---|---|---|
| The Hague | 22 January 2015 | Parry, Julian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 14 16 5140

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-16(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
# SHEET B

Application Number
EP 14 16 5140

The Search Division considers that the present European patent application does not comply with the requirements of unity of invention and relates to several inventions or groups of inventions, namely:

1. claims: 1, 2, 4, 7-16(all partially)

The subject matter of claims 1-2,4,7-16 insofar as they may relate to the case where R5 is a linear or branched C1-C6 alkyl group; R5' is a linear or branched C1-C6 alkyl group; R6 is (CR9)3 and R6' is (CR9)3.

---

2. claims: 1, 2, 4, 7-16(all partially)

The subject matter of claims 1-2,4,7-16 insofar as they may relate to the case where R5 is a linear or branched C1-C6 alkyl group; R5' is OR; R6 is (CR9)3 and R6' is (CR9)3.

---

3. claims: 1, 2, 4, 7-16(all partially)

The subject matter of claims 1-2,4,7-16 insofar as they may relate to the case where R5 is OR and R5' is OR; R6 is (CR9)3 and R6' is (CR9)3.

---

4. claims: 1-16(partially)

The subject matter of claims 1-16 insofar as they may relate to the case where R5' is a linear or branched C1-C6 alkyl group; R5 and R6 form together an unsubstituted 4-7 membered ring.

---

5. claims: 1-16(partially)

The subject matter of claims 1-16 insofar as they may relate to the case where R5' is OR; R5 and R6 form together an unsubstituted 4-7 membered ring.

---

6. claims: 1-16(partially)

The subject matter of claims 1-16 insofar as they may relate to the case where R5 is a linear or branched C1-C6 alkyl group; R5' and R6' form together an unsubstituted 4-7 membered ring.

---

7. claims: 1-16(partially)

The subject matter of claims 1-16 insofar as they may relate to the case where R5 is OR; R5' and R6' form together an unsubstituted 4-7 membered ring.

---

Europäisches Patentamt
European Patent Office
Office européen des brevets

# LACK OF UNITY OF INVENTION SHEET B

Application Number
EP 14 16 5140

The Search Division considers that the present European patent application does not comply with the requirements of unity of invention and relates to several inventions or groups of inventions, namely:

8. claims: 1-16(partially)

The subject matter of claims 1-16 insofar as they may relate to the case where R5 and R6 and R5' and R6' form together an unsubstituted 4-7 membered ring.

---

## ANNEX TO THE EUROPEAN SEARCH REPORT ON EUROPEAN PATENT APPLICATION NO. EP 14 16 5140

This annex lists the patent family members relating to the patent documents cited in the above-mentioned European search report. The members are as contained in the European Patent Office EDP file on
The European Patent Office is in no way liable for these particulars which are merely given for the purpose of information.

22-01-2015

| Patent document cited in search report | Publication date | Patent family member(s) | Publication date |
|---|---|---|---|
| WO 2006065844 A2 | 22-06-2006 | AT 458742 T | 15-03-2010 |
| | | EP 1833858 A2 | 19-09-2007 |
| | | EP 1833859 A2 | 19-09-2007 |
| | | EP 1866322 A2 | 19-12-2007 |
| | | US 2006160967 A1 | 20-07-2006 |
| | | US 2006160968 A1 | 20-07-2006 |
| | | US 2006183874 A1 | 17-08-2006 |
| | | US 2010113717 A1 | 06-05-2010 |
| | | WO 2006065809 A2 | 22-06-2006 |
| | | WO 2006065843 A2 | 22-06-2006 |
| | | WO 2006065844 A2 | 22-06-2006 |
| | | WO 2006065906 A2 | 22-06-2006 |
| WO 2012001052 A2 | 05-01-2012 | CA 2803499 A1 | 05-01-2012 |
| | | CN 102985434 A | 20-03-2013 |
| | | EP 2402353 A1 | 04-01-2012 |
| | | KR 20130031912 A | 29-03-2013 |
| | | US 2013289229 A1 | 31-10-2013 |
| | | WO 2012001052 A2 | 05-01-2012 |
| WO 2013029699 A1 | 07-03-2013 | CN 103930481 A | 16-07-2014 |
| | | EP 2562215 A1 | 27-02-2013 |
| | | WO 2013029699 A1 | 07-03-2013 |
| EP 2657285 A1 | 30-10-2013 | NONE | |
| WO 2012084961 A1 | 28-06-2012 | CN 103380151 A | 30-10-2013 |
| | | EP 2655431 A1 | 30-10-2013 |
| | | JP 2014505136 A | 27-02-2014 |
| | | KR 20140007360 A | 17-01-2014 |
| | | US 2014018506 A1 | 16-01-2014 |
| | | WO 2012084961 A1 | 28-06-2012 |
| WO 2011135005 A2 | 03-11-2011 | CN 103025767 A | 03-04-2013 |
| | | EP 2563821 A2 | 06-03-2013 |
| | | JP 2013525558 A | 20-06-2013 |
| | | KR 20130060208 A | 07-06-2013 |
| | | US 2013131291 A1 | 23-05-2013 |
| | | WO 2011135005 A2 | 03-11-2011 |
| WO 2011135004 A2 | 03-11-2011 | CN 102947354 A | 27-02-2013 |
| | | EP 2383299 A1 | 02-11-2011 |
| | | JP 2013525557 A | 20-06-2013 |
| | | KR 20130040893 A | 24-04-2013 |
| | | US 2013116394 A1 | 09-05-2013 |
| | | WO 2011135004 A2 | 03-11-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 16 5140

This annex lists the patent family members relating to the patent documents cited in the above-mentioned European search report. The members are as contained in the European Patent Office EDP file on
The European Patent Office is in no way liable for these particulars which are merely given for the purpose of information.

22-01-2015

| Patent document cited in search report | Publication date | Patent family member(s) | Publication date |
|---|---|---|---|
| WO 2007116034 A1 | 18-10-2007 | EP 2004664 A1 | 24-12-2008 |
| | | JP 2009533382 A | 17-09-2009 |
| | | US 2009275712 A1 | 05-11-2009 |
| | | WO 2007116034 A1 | 18-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007116034 A
- WO 2007122098 A
- EP 2532687 A
- WO 2011135004 A
- WO 2003051934 A
- WO 2012075560 A
- WO 2003102042 A
- WO 200202576 A
- WO 2012084961 A
- WO 2012001052 A
- WO 2011076780 A
- WO 03051934 A
- WO 2006069733 A
- WO 2010052263 A
- WO 2010052260 A
- WO 2010052264 A
- WO 2012001051 A1
- WO 2013007650 A


### Non-patent literature cited in the description


- **KLIMKE, K. ; PARKINSON, M. ; PIEL, C. ; KAMINSKY, W. ; SPIESS, H.W. ; WILHELM, M.** *Macromol. Chem. Phys.,* 2006, vol. 207, 382
- **PARKINSON, M. ; KLIMKE, K. ; SPIESS, H.W. ; WILHELM, M.** *Macromol. Chem. Phys.,* 2007, vol. 208, 2128
- NMR Spectroscopy of Polymers: Innovative Strategies for Complex Macromolecules. 2011, 401
- **POLLARD, M. ; KLIMKE, K. ; GRAF, R. ; SPIESS, H.W. ; WILHELM, M. ; SPERBER, O. ; PIEL, C. ; KAMINSKY, W.** *Macromolecules,* 2004, vol. 37, 813
- **FILIP, X. ; TRIPON, C. ; FILIP, C.** *J. Mag. Resn.,* 2005, vol. 176, 239
- **GRIFFIN, J.M. ; TRIPON, C. ; SAMOSON, A. ; FILIP, C. ; BROWN, S.P.** *Mag. Res. in Chem.,* 2007, vol. 45 (S1), S198
- **CASTIGNOLLES, P. ; GRAF, R. ; PARKINSON, M. ; WILHELM, M. ; GABORIEAU, M.** *Polymer,* 2009, vol. 50, 2373
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 1128
- **J. RANDALL.** *Macromol. Sci., Rev. Macromol. Chem. Phys.,* 1989, vol. C29, 201
- **QIU, X. ; REDWINE, D. ; GOBBI, G. ; NUAMTHANOM, A. ; RINALDI, P.** *Macromolecules,* 2007, vol. 40, 6879
- **LIU, W. ; RINALDI, P. ; MCINTOSH, L. ; QUIRK, P.** *Macromolecules,* 2001, vol. 34, 4757
- **STORK, G. ; WHITE, W. N.** *J. Am. Chem. Soc.,* 1956, vol. 78, 4604
- **HINTERMANN, L.** *Beilstein J. Org. Chem.,* 2007, vol. 3, 1
- **UENO, K. ; SHIBATA, Y.** *Organometallics,* 2006, vol. 25, 3422